# EUROPÄISCHE PATENTSCHRIFT

(11) **EP 1 272 453 B1**
(45) Veröffentlichungstag und Bekanntmachung des Hinweises auf die Patenterteilung: **09.11.2005**
(21) Anmeldenummer: 01919426.5
(22) Anmeldetag: 04.04.2001
(51) Int. Cl.: C07C 51/43, C07C 51/47, C07C 57/07

(54) **VERFAHREN ZUR REINIGUNG EINER ROHACRYLSÄURESCHMELZE**
METHOD FOR THE PURIFICATION OF A CRUDE ACRYLIC ACID MELT
PROCEDE DE PURIFICATION D'UNE MASSE FONDUE D'ACIDE ACRYLIQUE BRUT

(30) Priorität: 11.04.2000 DE 10017903; 28.07.2000 DE 10036881; 28.07.2000 DE 10036880; 10.08.2000 DE 10039025
(43) Veröffentlichungstag der Anmeldung: 08.01.2003
(73) Patentinhaber: BASF Aktiengesellschaft, 67056 Ludwigshafen (DE)
(72) Erfinder: ECK, Bernd, 68519 Viernheim (DE); BAUMANN, Dieter, 67227 Frankenthal (DE); HEILEK, Jörg, 69245 Bammental (DE); MÜLLER-ENGEL, Klaus, Joachim, 76297 Stutensee (DE)
(86) Internationale Anmeldenummer: PCT/EP2001/003827
(87) Internationale Veröffentlichungsnummer: WO 2001/077056

(56) Entgegenhaltungen:
- WO-A-98/25889
- DE-A- 19 833 049
- DE-A- 19 924 533

## Beschreibung

Vorliegende Erfindung betrifft ein Verfahren zur Reinigung einer Rohacrylsäureschmelze, die, bezogen auf ihr Gewicht, in das in der Rohacrylsäureschmelze enthaltenes Wasser nicht mit eingerechnet wird,
≥ 80 Gew.-% Acrylsäure
und als von Acrylsäure verschiedene Verunreinigungen wenigstens
≥ 100 Gew.-ppm Essigsäure und
≥ 10 Gew.-ppm Propionsäure
enthält, bei dem man die Rohacrylsäureschmelze unter Einwirkung von Kälte in eine aus Acrylsäurekristallen und Restschmelze bestehende Rohacrylsäuresuspension überführt, wobei der Gewichtsanteil der Acrylsäurekristalle an von Acrylsäure verschiedenen Verunreinigungen kleiner und der Gewichtsanteil der Restschmelze an von Acrylsäure verschiedenen Verunreinigungen größer ist als der Gewichtsanteil der Rohacrylsäureschmelze an von Acrylsäure verschiedenen Verunreinigungen, von der Rohacrylsäuresuspension gegebenenfalls einen Teil der Restschmelze mechanisch abtrennt und die Acrylsäurekristalle der verbliebenen Rohacrylsäuresuspension in einer Waschkolonne von verbliebener Restschmelze befreit.

Acrylsäure, entweder für sich oder in Form ihrer Salze oder ihrer Ester, ist insbesondere zur Herstellung von Polymerisaten für die verschiedensten Anwendungsgebiete (z.B. Klebstoffe, Superabsorber, Bindemittel) von Bedeutung.

Unter anderem ist Arylsäure durch katalytische Gasphasenoxidation von Propan, Propen und/oder Acrolein erhältlich. Dabei werden diese Ausgangsgase, in der Regel mit inerten Gasen wie Stickstoff, CO₂ und/oder Wasserdampf verdünnt, im Gemisch mit Sauerstoff bei erhöhten Temperaturen sowie gegebenenfalls erhöhtem Druck über übergangsmetallische Mischoxidkatalysatoren geleitet und oxidativ in ein Acrylsäure enthaltendes Produktgemisch umgewandelt. Durch Kondensation des Produktgemisches oder durch Aufnahme in ein geeignetes Absorptionsmittel (z.B. Wasser oder ein Gemisch aus 70 bis 75 Gew.-% Diphenylether und 25 bis 30 Gew.-% Diphenyl) kann eine Grundabtrennung der Acrylsäure aus dem Produktgasstrom erzielt werden (vgl. z.B. EP-A 297 445 und DE-PS 2 136 396).

Durch Entfernung des Absorptionsmittels (und gegebenenfalls zuvor erfolgte Desorption von eine geringe Absorptionsmittellöslichkeit aufweisende Verunreinigungen durch Abstreifen, z.B. mit Luft) über extraktive und/oder destillative Trennverfahren (z.B. Entfernung des Absorptionsmittels Wasser durch Destillation, azeotrope Destillation oder extraktive Abtrennung der Säure aus der wäßrigen Lösung und anschließende destillative Entfernung des Extraktionsmittels) und/oder nach Anwendung von sonstigen Trennschritten wird häufig eine Acrylsäure erhalten, die hier als Rohacrylsäure bezeichnet wird.

Diese Rohacrylsäure ist kein reines Produkt. Vielmehr enthält sie ein Spektrum verschiedener, für den gasphasenkatalytisch oxidativen Herstellungsweg typischer, Verunreinigungen. Diese sind insbesondere Propionsäure, Essigsäure und niedermolekulare Aldehyde (in typischer Weise beträgt der Gesamtgehalt an niedermolekularen Aldehyden in erfindungsgemäß zu behandelnder Rohacrylsäure ≥ 100 Gew.-ppm, bezogen auf das Gewicht von wasserfrei gerechneter Rohacrylsäure; in der Regel liegt der vorgenannte Aldehydgehalt bei Werten ≤ 1 Gew.-%) wie Acrolein, Methacrolein, Propionaldehyd, n-Butyraldehyd, Benzaldehyd, Furfurale und Crotonaldehyd. Je nach Herstellungsweg der Rohacrylsäure kann sie als weitere Verunreinigung auch Wasser enthalten. Ein weiterer typischer Bestandteil von Rohacrylsäuren sind Polymerisationsinhibitoren. Diese werden im Verlauf der zur Herstellung von Rohacrylsäure angewandten Trennprozesse zugesetzt, wo sie eine mögliche radikalische Polymerisation der α,β-monoethylenisch ungesättigten Acrylsäure unterdrücken sollen, weshalb sie auch als Prozeßstabilisatoren bezeichnet werden. Eine herausragende Position unter den Acrylsäure-Prozeßstabilisatoren nehmen Dibenzo-1,4-thiazin (PTZ), 4-Hydroxy-2,2,6,6-tetramethylpiperidin-1-oxyl(4-OH-TEMPO) und p-Methoxyphenol (MEHQ) ein, die entweder jeweils für sich oder paarweise oder als Dreiergemisch Bestandteil von Rohacrylsäure sein können. Üblicherweise beträgt die Gesamtmenge an in Rohacrylsäure enthaltenen Polymerisationsinhibitoren, bezogen auf das Gewicht der Rohacrylsäure (wobei in der Rohacrylsäure enthaltenes Wasser nicht miteingerechnet wird) 0,1 bis 2 Gew.-%.

Weitere unerwünschte Begleiter von in kondensierter Phase befindlicher Acrylsäure sind die durch Michael-Addition von Acrylsäure an sich selbst sowie an das sich dabei bildende Acrylsäure-Dimere entstehenden Acrylsäure-Oligomere (Michael-Addukte). Während diese Verbindungen in frisch erzeugter Rohacrylsäure normalerweise so gut wie nicht enthalten sind (üblicherweise beträgt ihr Gewichtsanteil bezogen auf das wasserfrei gerechnete Gewicht der Rohacrylsäure < 0,01 Gew.-%), entstehen sie in selbiger bei längerem sich selbst überlassen. Aus statistischen Gründen ist dabei die Bildung von Diacrylsäure von besonderer Bedeutung, wohingegen die Bildung höherer Acrylsäure-Oligomere (Trimere, Tetramere etc.) nur in einer untergeordneten Menge erfolgt.

Die Gesamtmenge an sonstigen in der Rohacrylsäure möglicherweise enthaltenen Nebenkomponenten beträgt auf das wasserfrei gerechnete Gewicht der Rohacrylsäure in der Regel nicht mehr als 10 Gew.-%.

Unter Rohacrylsäure soll in dieser Schrift deshalb insbesondere solche Rohacrylsäure verstanden werden, die, bezogen auf ihr wasserfrei gerechnetes Gewicht,
≥ 80 Gew.-% Acrylsäure,
≥ 100 Gew.-ppm bis ≤ 15 Gew-% Essigsäure,
≥ 10 Gew.-ppm bis ≤ 5 Gew-% Propionsäure,
bis zu 5 Gew.-% niedermolekulare Aldehyde,
bis zu 3 Gew.-% Polymerisationsinhibitoren und
0 bis 5 Gew.-% Acrylsäure-Oligomere (Michael-Addukte)
enthält.

Damit umfaßt der hier verwendete Begriff Rohacrylsäure vor allem auch solche Rohacrylsäure, die, bezogen auf ihr wasserfrei gerechnetes Gewicht,
≥ 90 Gew.-% Acrylsäure,
≥ 100 Gew.-ppm bis ≤ 5 Gew-% Essigsäure,
≥ 10 Gew.-ppm bis ≤ 2 Gew-% Propionsäure,
bis zu 2 Gew.-% niedermolekularen Aldehyde,
bis zu 2 Gew.-% Polymerisationsinhibitoren und
0 bis 3 Gew.-% Acrylsäure-Oligomere (Michael-Addukte) enthält.

Nicht zuletzt umfaßt der hier verwendete Begriff Rohacrylsäure damit solche Rohacrylsäure, die, bezogen auf ihr wasserfrei gerechnetes Gewicht,
≥ 95 Gew.-% Acrylsäure,
≥ 100 Gew.-ppm bis ≤ 3 Gew-% Essigsäure,
≥ 10 Gew.-ppm bis ≤ 2 Gew-% Propionsäure
bis zu 2 Gew.-% niedermolekularen Aldehyde,
bis zu 2 Gew.-% Polymerisationsinhibitoren und
0 bis 2 Gew.-% Acrylsäure-Oligomere (Michael-Addukte)
enthält.

Bezogen auf die in der Rohacrylsäure enthaltene Menge an Acrylsäure enthalten die Rohacrylsäuren häufig kein Wasser, teilweise aber auch bis zu 5 Gew.-% oder bis zu 4 Gew.-% oder bis zu 3 Gew.-% an Wasser.

Von den in den vorgenannten Rohacrylsäuren neben Acrylsäure enthaltenen Bestandteilen erweisen sich die meisten im Rahmen einer Rohacrylsäureverwendung als nachteilig.

Würde eine solche Rohacrylsäure beispielsweise zur Herstellung von Estern aus C₁-C₈-Alkanolen und Acrylsäure verwendet, würden in Nebenreaktionen auch die entsprechenden Essigsäure- und Propionsäureester gebildet, was die Ausbeute an gewünschtem Acrylsäureester, bezogen auf die eingesetzte Menge an Alkanol, mindert. Setzt man die im Beisein der niedermolekularen Aldehyde gebildeten Acrylsäureester zu radikalischen Polymerisationen ein, wirkt sich deren Gehalt an den niedermolekularen Aldehyden in der Regel z.B. insofern nachteilig aus, als sie z.B. die Induktionszeit von Polymerisationsreaktionen, d.h., den Zeitraum zwischen dem Erreichen der Polymerisationstemperatur und dem tatsächlichen Beginn der Polymerisation, beeinflussen. Ferner beeinflussen sie in der Regel den Polymerisationsgrad und können in den Polymerisaten auch Verfärbungen verursachen.

Vorgenannte Nachteile treffen normalerweise auch dann zu, wenn man die Rohacrylsäure unmittelbar als Acrylsäurequelle in Polymerisationen anwendet.

Zur Herstellung von Superabsorbern (= Massen zur Aufnahme von Wasser auf der Grundlage von Polyacrylsäure und deren Salzen) zu verwendende Acrylsäurequellen unterliegen insbesondere dem Erfordernis, möglichst keine Diacrylsäure und möglichst kein Dibenzo-1,4-thiazin enthalten zu dürfen, da beide Bestandteile entweder bei der Superabsorberherstellung (insbesondere Dibenzo-1,4-thiazin stört aufgrund seiner radikalische Polymerisationen extrem inhibierenden Wirkung bei der Herstellung von Superabsorbern empfindlich) oder beim Superabsorbergebrauch (Superabsorber finden insbesondere im Hygienebereich Verwendung (z.B. Babywindeln); der Abschluß der Superabsorberherstellung besteht in der Regel aus einer Hochtemperaturoberflächenvernetzung; bei den angewandten Vernetzungstemperaturen wird copolymerisierte Diacrylsäure unter Ausbildung von monomerer Acrylsäure wenigstens partiell rückgespalten (inverse Michael-Addition); ein erhöhter Gehalt an monomerer Acrylsäure ist in diesem Anwendungssektor jedoch nicht tolerabel) unerwünscht sind.

In der Praxis werden zur Weiterreinigung von Rohacrylsäure insbesondere rektifikative Trennoperationen angewendet (vgl. z.B. EP-A 722 926).

Nachteilig an diesen Trennverfahren ist, daß sie insbesondere zur Abtrennung der ähnlich wie Acrylsäure siedenden Bestandteile (z.B. Propionsäure) einen hohen Energieaufwand erfordern, da entweder unter hohen Rücklaufverhältnissen und/oder mit eine Vielzahl an theoretische Trennstufen aufweisenden Rektifikationskolonnen gearbeitet werden muß. Deshalb wurde z.B. auch schon versucht, durch geeignete Gestaltung des Gasphasenoxidationsverfahrens an Propion- und/oder Essigsäure freie Acrylsäure zu synthetisieren (vgl. z.B. JP-A 11-35519 und EP-A 253409). Darüber hinaus bedingt die thermische Beanspruchung bei rektifikativen Reinigungsverfahren in der Regel unerwünschte radikalische Polymerisation der Acrylsäure.

Als Alternative findet in jüngster Vergangenheit zunehmend die Verfahrensweise der Schmelzkristallisation zur Reinherstellung von Acrylsäure Interesse (vgl. z.B. EP-A 616 998). Ganz generell wird dabei die verunreinigte Rohacrylsäure(schmelze) durch Abkühlen partiell erstarrt. Entsprechend dem Phasengleichgewicht weisen die entstehenden Acrylsäurekristalle einen geringeren Verunreinigungsgehalt auf als die zurückbleibende flüssige Restschmelze. Abgeschwächt wird der vorstehend beschriebene, rein thermodynamisch bestimmte Trenneffekt durch Einschluß von Flüssigkeit während des Kristallisationsvorganges und durch die dem Feststoff nach einer Fest/Flüssig-Trennung noch anhaftende Restschmelze. Für das Erreichen hoher Reinheiten sind daher selbst bei eutektischen Systemen oftmals mehrere aufeinanderfolgende Kristallisationsstufen erforderlich. D. h., die in einer ersten Kristallisationsstufe gewonnenen Kristalle werden wieder aufgeschmolzen und einem neuen Kristallisationsschritt unterworfen u.s.w.. Nachteilig an einer solchen taktweisen Verfahrensweise ist, daß in jeder Stufe die Kristallisationswärme beim Ausfrieren ab- und beim anschließenden Aufschmelzen wieder zugeführt werden muß. Dies beeinträchtigt die Wirtschaftlichkeit kristallisativer Trennverfahren. Für eine wirtschaftliche Anwendung von Schmelzkristallisationsverfahren ist es deshalb von ganz entscheidender Bedeutung, mit möglichst wenigen Kristallisationsstufen eine möglichst hohe Reinheit der abgetrennten Kristalle zu erzielen.

Zur kristallisativen Reinigung von Rohacrylsäureschmelzen empfiehlt der Stand der Technik überwiegend Schichtkristallisationsverfahren (vgl. z.B. DE-OS 2 606 364, EP-A 616 998, EP-A 648 520 und EP-A 776875).

Bei den Schichtkristallisationsverfahren wird das Kristallisat in Form zusammenhängender, fest anhaftender Schichten ausgefroren. Die Fest/Flüssig-Trennung erfolgt durch einfaches Abfließen lassen der Restschmelze. Anschließend wird das gereinigte Kristallisat aufgeschmolzen. Prinzipiell unterscheidet man zwischen "statischen" und "dynamischen" Schichtkristallisationsverfahren.

Bei den statischen Verfahren wird die zu reinigende Rohacrylsäureschmelze z.B. in Rohrbündel- oder modifizierte Plattenwärmeaustauscher eingefüllt und anschließend durch langsame Temperatursenkung auf der Sekundärseite teilweise erstarrt. Nach dem Ausfrieren wird die Restschmelze abgelassen und durch langsame Temperturerhöhung anschließend zunächst stärker, später weniger stark verunreinigte Fraktionen aus der Kristallschicht abgeschmolzen, bis schließlich Produkt mit hoher Reinheit abgeschmolzen wird. Dieser Vorgang wird in der Literatur mit Schwitzen bezeichnet. Statische Kristallisationsverfahren erzielen bei Rohacrylsäuren zwar in einer Kristallisationsstufe eine signifikante Reinigungswirkung. Von Nachteil ist jedoch die bei statischen Kristallisationsverfahren üblicherweise geringe Raum-Zeit-Ausbeute, da bei statischen Schmelzkristallisationen der Wärme- und Stofftransport zu den Abscheideflächen nur durch freie Konvektion erfolgt.

Kennzeichnend für die dynamische Schichtkristallisation von Rohacrylsäureschmelzen ist eine erzwungene Konvektion der Rohacrylsäureschmelze. Diese kann durch Umpumpen der Rohacrylsäureschmelze durch volldurchströmte Rohre (z.B. DE-OS 2 606 364), durch Aufgabe der Rohacrylsäureschmelze als Rieselfilm (z.B. EP-A 616 998) oder durch Einleiten von Inertgas in ein mit Schmelze gefülltes Rohr oder durch Pulsieren erfolgen.

Nachteilig an einer dynamischen Schichtkristallisationsreinigung von Rohacrylsäureschmelzen ist, daß die Reinigungswirkung innerhalb einer Kristallisationsstufe bei erhöhten Verunreinigungsgehalten der Rohacrylsäureschmelze nicht zu befriedigen vermag, weshalb die EP-A 616 998 zur Reinigung von Rohacrylsäureschmelzen die Anwendung einer Kombination von statischer und dynamischer Schichtkristallisation empfiehlt. Nachteilig an dieser Verfahrensweise ist jedoch, daß sie in notwendiger Weise mehrerer Kristallisationsstufen bedarf. Eine gewisse Verbesserung kann zwar durch Anwendung der in der DE-A 3 708 709 empfohlenen Waschung der abgeschiedenen Kristallschichten mit reineren Schmelzfraktionen bewirkt werden. Aufgrund der geringen spezifischen Oberfläche der abgeschiedenen Schichten vermag der Wascheffekt jedoch nicht in vollem Umfang zu befriedigen.

Die EP-A 616 998 bezieht zur kristallisativen Reinigung von Rohacrylsäureschmelze zwar die Möglichkeit einer Suspensionskristallisation mit ein, eine Waschung der abgetrennten Suspensionskristalle von anhaftender Restschmelze wird jedoch nicht in Betracht gezogen. Statt dessen wird eine Kombination mit statischen Kristallisationsstufen empfohlen, was wegen der damit in notwendiger Weise einhergehenden Mehrstufigkeit der Verfahrensweise nicht zu befriedigen vermag.

Bei den Suspensionskristallisationsverfahren wird in der Regel durch Kühlung der die Verunreinigungen enthaltenden Ausgangsschmelze eine Kristallsuspension erzeugt, die aus einen geringeren Verunreinigungsgehalt aufweisenden Kristallen und einen höheren Verunreinigungsgehalt aufweisender Restschmelze besteht. Dabei können die Feststoffkristalle unmittelbar in Suspenison befindlich wachsen oder sich als Schicht auf einer gekühlten Wand abscheiden, von der sie anschließend abgekratzt und in der Restschmelze resuspendiert werden. D.h., die Feststoffbildung kann in gekühlten Rührkesseln, in Kratzkühlern oder in Scheibenkristallisatoren durchgeführt werden, wie sie z.B. in Chem.-Ing.-Techn. 57 (1985) Nr.2, S.91-102 beschrieben sind.

Die anschließend erforderliche Abtrennung der Restschmelze vom Kristallisat kann zunächst rein mechanisch durch Abpressen, Filtrieren und/oder Zentrifugieren erfolgen (vgl. z.B. Chem.-Ing.-Techn. 57 (1985) Nr.2, S.91-102).

Nachteilig an einer solchen Verfahrensweise mit rein mechanischer Trennung von Kristallisat und Restschmelze ist, daß aufgrund der am Kristallisat haftend verbleibenden Restschmelze die in einem Trennschritt erzielbare Reinigungswirkung im Fall von Rohacrylsäureschmelzen nicht zu befriedigen vermag.

In der älteren Anmeldung DE-A 19926082 wird daher empfohlen, das mechanisch abgetrennte Acrylsäuresuspensionskristallisat nachträglich zusätzlich mit einer Acrylsäure enthaltenden Waschflüssigkeit zu waschen, wobei als Waschflüssigkeit vorzugsweise eine Acrylsäureschmelze verwendet wird, deren Gewichtsanteil an von Acrylsäure verschiedenen Verunreinigungen geringer als der entsprechende Verunreinigungsgehalt das zu waschenden, mechanisch abgetrennten Suspensionskristallisates ist.

Nachteilig an der in der DE-A 19926082 angewandten Waschweise ist, daß ihre Reinigungswirkung nicht in vollem Umfang zu befriedigen vermag. Dies ist vermutlich insbesondere darauf zurückzuführen, daß der bewirkte Kontakt zwischen zu waschenden Kristallen und Waschflüssigkeit nicht voll zu befriedigen vermag.

Es ist nun allgemein bekannt, daß bei einem Suspensionskristallisatbrei eine Trennung von Suspensionskristallisat und Restschmelze auch entweder ausschließlich oder nach einer mechanisch erfolgten Teilabtrennung (insbesondere vorab einer Anwendung einer mechanischen Waschkolonne) von Restschmelze mittels einer geeigneten Waschflüssigkeit in einer sogenannten Waschkolonne vorgenommen werden kann, in der die Waschflüssigkeit zu den Suspensionskristallen im Gegenstrom geführt wird.

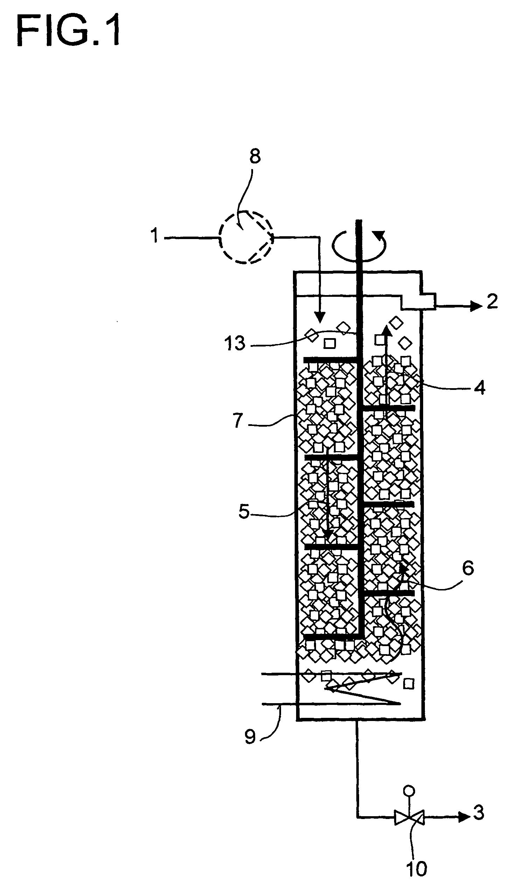

Prinzipiell unterscheidet man (vgl. Fig. 1 bis 4) die Waschkolonnentypen in solche mit erzwungenem Transport des Suspensionskristallbetts und solche mit einem Schwerkraft-Transport der Suspensionskristalle (eine ausführliche Beschreibung der unterschiedlichen Waschkolonnentypen findet sich u.a. in Chem.-Ing.-Techn. 57(1985) Nr. 291-102, in Chemical Engineering Science Bd.50, Nr.17, S.2712 bis 2729, 1995, Elsevier Science Ltd., in Applied Thermal Engineering Bd.17, Nr.8-10, S.879-888, 1997, Verlag Elsevier Science Ltd. und den in den vorgenannten Literaturstellen aufgeführten Literaturzitaten). Bei Waschkolonnen mit erzwungenem Transport des Suspensionskristallbetts wird wenigstens eine von der Gravitation verschiedene in die Transportrichtung wirkende Kraft zum Transport des Suspensionskristallbetts angewendet.

Innerhalb der Waschkolonne werden die Suspensionskristalle entweder von oben nach unten oder von unten nach oben transportiert. Die Waschflüssigkeit wird in der Waschkolonne im Gegenstrom zu den Suspensionskristallen geführt. In den älteren Schriften DE-A 19626839, DE-A 19740252, DE-A 19829477, DE-A 19832962, DE-A 19833049 und DE-A 19838845 wird als zu verwendende Waschflüssigkeit für Rohacrylsäuresuspensionen u.a. Wasser oder wäßrige Acrylsäure empfohlen. Nachteilig an diesen Waschflüssigkeiten ist jedoch, daß einerseits ihre Reinigungswirkung nicht voll zu befriedigen vermag und sie andererseits erhebliche Acrylsäureverluste bedingen.

Alternativ zur vorgenannten Verfahrensweise kann man auch die am Ende ihrer Transportstrecke in der Waschkolonne gereinigt ankommenden Suspensionskristalle aufschmelzen (am gegenüberliegenden Teil der Waschkolonne wird in der Regel die Mutterlauge entnommen), nur eine Teilmenge der dadurch anfallenden gereinigten Schmelze entnehmen und die Restmenge der gereinigten Schmelze als Waschschmelze in der bzw. in die Waschkolonne rückführen und zwar im Gegenstrom zu den der Waschkolonne zugeführten Suspensionskristallen (in dieser Schrift sollen solchermaßen betriebene Waschkolonnen im engeren Sinn als Waschschmelze- Waschkolonnen bezeichnet werden). Dabei kann es je nach materieller Beschaffenheit der in der Waschkolonne zu behandelnden Kristallsuspension entweder aufgrund aller oder aber nur aufgrund einiger der verschiedenen nachfolgend aufgelisteten Mechanismen zu einer Reinigungswirkung kommen:
- Verdrängen der Restschmelze (Mutterlauge) durch die Waschschmelze,
- Abwaschen der an den Suspensionskristallen anhaftenden Schicht Restschmelze durch die Waschschmelze,
- Diffusionswäsche von wenig/nicht durchströmten Bereichen zwischen (z.B. großflächig aufeinanderliegenden) Suspensionskristallen mit Waschschmelze,
- Auskristallisieren der in die Waschkolonne rückgeführten Waschschmelze an den im Gegenstrom geführten Suspensionskristallen,
- Schwitzen der Suspensionskristalle im Kontakt mit der Waschschmelze,
- adiabatisches Umkristallisieren der Suspensionskristalle im Kontakt mit der Waschschmelze.

Die letzten drei der vorgenannten Reinigungsmechanismen sollen hier als "Extrareinigungsmechanismen" bezeichnet werden.

Gemäß Chemical Engineering Science Bd.50, Nr.17, S.2717-2729, 1995, Elsevier Science Ltd. hängt der Beitrag der individuellen Reinigungsmechanismen u.a. von der Kontaktzeit zwischen den Suspensionskristallen und der Waschschmelze sowie von der Morphologie und Zusammensetzung der Suspensionskristalle ab. Prinzipiell kann keiner der vorgenannten Reinigungsmechanismen ausgeschlossen werden, weil die Schmelztemperatur der gereinigten Kristalle infolge der geringeren Schmelzpunkterniedrigung durch Verunreinigungen höher liegt als die Temperatur der noch nicht "gewaschenen" Kristalle, die im wesentlichen der Gleichgewichtstemperatur der Rohacrylsäuresuspension entspricht.

Bei Schwerkraft-Waschschmelze-Waschkolonnen erfolgt der Transport der Suspensionskristalle längs der Waschkolonne durch die Schwerkraft gegen die eine geringere Massendichte aufweisende und deshalb spezifisch leichtere (und daher in der Waschkolonne aufsteigende) Waschschmelze. Ein sich langsam drehender Rührer (üblicherweise < 0,035 Umdrehungen je Sekunde) erstreckt sich häufig über die gesamte Schwerkraft-Waschschmelze-Waschkolonne und dient dazu, Agglomeration und/oder Kanalbildung im sich abwärts bewegenden Kristallbett zu verhindern. Die Verweilzeit der Suspensionskristalle in einer Schwerkraft-Waschschmelze-Waschkolonne beträgt ≥ 1 h (der Unterschied in der Massendichte zwischen flüssiger und fester Phase ist in der Regel ≤ 15 %). Ferner beträgt der geringste Porositätsgrad innerhalb des Kristallbetts in einer Schwerkraft-Waschschmelze-Waschkolonne normalerweise > 0,45, oft ≤ 0,65. Die Mutterlauge verläßt die Schwerkraft-Waschschmelze-Waschkolonne normalerweise über einen Überlauf. Der Vorteil einer Schwerkraft-Waschschmelze-Waschkolonne besteht darin, daß ihre langen Verweilzeiten der Kristalle im besonderen Umfang die Extrareinigungsmechanismen zur Anwendung bringen. Ein Schwachpunkt der Schwerkraft-Waschschmelze-Waschkolonne besteht gemäß Applied Thermal Engineering Bd.17, Nr.8-10, S.879-888, 1997, Elsevier Science Ltd. im Bedarf relativ großer Kristalle.

Bei Waschschmelze-Waschkolonnen mit erzwungenem Transport des Suspensionskristallbetts unterscheidet man z.B. Druckkolonnen (auch Hydraulikkolonnen genannt), bei denen der Transport der Kristalle und der Waschschmelze z.B. durch Pumpen und/oder hydrostatische Höhe von außen bewirkt und die Mutterlauge in der Regel über Filter aus der Waschkolonne gepreßt wird (jenseits der Filter kann Normaldruck, Unterdruck oder überatmosphärischer Druck herrschen), von mechanischen Kolonnen mit mechanischen Zwangsfördereinrichtungen für das Kristallbett wie speziellen Stempelpressen, Rührern, Schnecken, Wendeln oder Spiralen. Mechanische Waschschmelze-Waschkolonnen eigenen sich insbesondere für die Reinigung von an Restschmelze armen Kristallsuspensionen. Die Mutterlauge wird in mechanischen Waschschmelze - Waschkolonnen in der Regel ebenfalls über Filter entnommen, die sich entweder hinter oder in der mechanischen Zwangsfördereinrichtung befinden.

Waschschmelze-Waschkolonnen mit erzwungenem Transport des Kristallbetts sind durch sehr viel kürzere Verweilzeiten der Kristalle in der Waschkolonne als im Fall einer Schwerkraft-Waschschmelze-Waschkolonne gekennzeichnet. Sie beträgt ≤ 30 min. und liegt in der Regel bei 10 bis 15 min., häufig bei 2 bis 8 min. Ferner beträgt der geringste Porositätsgrad (= Porenvolumen/Gesamtvolumen) innerhalb des Kristallbetts einer Waschschmelze-Waschkolonne mit erzwungenem Transport normalerweise ≤ 0,45. Gemäß Chemical Engineering Science Bd. 50, Nr. 17, S. 2717-2729, 1995, Elserier Science Ltd. sind in Waschschmelze-Waschkolonnen mit erzwungenem Transport die Verweilzeiten der Kristalle zu kurz, um die Extra-Reinigungsmechanismen mit signifikanter Wahrscheinlichkeit eintreten zu lassen.

Aus der JP-A 7-82210 ist ein Verfahren zur kristallisativen Reinigung von Rohacrylsäure bekannt, bei der zunächst aus der Rohacrylsäureschmelze im Beisein von Wasser durch Einwirkung von Kälte eine Rohacrylsäuresuspension erzeugt wird, wobei das Beisein von Wasser den Zweck verfolgt, die erforderliche Kälte durch Verdampfungskühlung zu erzeugen. Lediglich beiläufig wird in der JP-A 7-82210 bemerkt, daß das Beisein des Wassers die Ausbildung der Acrylsäurekristalle dahingehend beeinflußt, daß besonders große Kristalle erwachsen.

Abschließend erfolgt in der JP-A 7-82210 eine Reinigungsbehandlung der erzeugten Rohacrylsäuresuspension mittels einer Schwerkraft-Waschschmelze-Waschkolonne. Die in der JP-A 7-82210 mit einer Reinigungsstufe erzielte Reinigungswirkung vermag zwar zu befriedigen, nicht jedoch die erzielte Raum-Zeit-Ausbeute. Vermerkt wurde in der JP-A 7-82210 auch, daß sich das Beisein des Wassers bei der Erzeugung der Rohacrylsäuresuspension auf die Reinheit der in der Schwerkraft-Waschschmelze-Waschkolonne gewaschenen Acrylsäurekristalle vorteilhaft auswirkt. Die Anwendung einer Schwerkraft-Waschschmelz-Waschkolonne wird auch in der EP-A 730893 empfohlen.

Die WO 99/06348 empfiehlt für eine befriedigende kristallisative Reinigung von Rohacrylsäure in einer Reinigungsstufe (insbesondere für eine befriedigende Abtrennung der Verunreinigungen Propionsäure und/oder Essigsäure) der Rohacrylsäure zunächst eine polare organische Substanz zuzusetzen, anschließend durch Einwirkung von Kälte eine Acrylsäuresuspension zu erzeugen und diese in einer mechanischen Waschschmelze-Waschkolonne zu waschen.

Nachteilig an dieser Verfahrensweise ist es, daß sie des Zusatzes eines polaren organischen Lösungsmittels zur Rohacrylsäure bedarf.

Aus einem Aufsatz von M. Nienoord, G.J. Arkenbout und D. Verdoes über "Experiences with the TNO-Hydraulic Wash Column" auf dem 4. BIWIC 94/Bremen International Workshop for Industrial Crystallization, Bremen, 8th - 9th September 1994 at the University Bremen, Ed.: J. Ulrich, ist bekannt, daß hydraulische Waschschmelze-Waschkolonnen prinzipiell zur Reinigung von Acrylsäuresuspensionen geeignet sind. Über die Zusammensetzung der Acrylsäuresuspension sowie über deren Herstellung enthält das vorgenannte Literaturzitat jedoch keine Angaben.

Angesichts des vorgenannten Standes der Technik bestand die Aufgabe der vorliegenden Erfindung darin, ein verbessertes Verfahren zur Reinigung von Rohacrylsäureschmelzen zur Verfügung zu stellen, das einerseits bereits in einer Reinigungsstufe Acrylsäuren in erhöhter Reinheit und mit hoher Raum-Zeit-Ausbeute zur Verfügung zu stellen vermag und andererseits insbesondere für eine befriedigende Abtrennung von Propion- und/oder Essigsäure keines Vorabzusatzes eines polaren organischen Lösungsmittels zur Rohacrylsäure bedarf.

Demgemäß wurde ein Verfahren zur Reinigung einer Rohacrylsäureschmelze, die, bezogen auf ihr Gewicht, in das in der Rohacrylsäureschmelze enthaltenes Wasser nicht mit eingerechnet wird,
≥ 80 Gew.-% Acrylsäure
und als von Acrylsäure verschiedene Verunreinigungen wenigstens
≥ 100 Gew.-ppm Essigsäure und
≥ 10 Gew.-ppm Propionsäure
enthält, bei dem man die Rohacrylsäureschmelze unter Einwirkung von Kälte in eine aus Acrylsäurekristallen und Restschmelze bestehende Rohacrylsäuresuspension überführt, wobei der Gewichtsanteil der Acrylsäurekristalle an von Acrylsäure verschiedenen Verunreinigungen kleiner und der Gewichtsanteil der Restschmelze an von Acrylsäure verschiedenen Verunreinigungen größer ist als der Gewichtsanteil der Rohacrylsäureschmelze an von Acrylsäure verschiedenen Verunreinigungen, von der Rohacrylsäuresuspension gegebenenfalls einen Teil der Restschmelze mechanisch abtrennt und die Acrylsäurekristalle der verbliebenen Rohacrylsäuresuspension in einer Waschkolonne von verbleibender Restschmelze befreit, gefunden, das dadurch gekennzeichnet ist, daß
a) die Erzeugung der Acrylsäurekristalle der Rohacrylsäuresuspension im Beisein von 0,20 bis 10 Gew.-%, bezogen auf das Gewicht der in der Rohacrylsäureschmelze enthaltenen Menge an Acrylsäure, an Wasser erfolgt,
b) die Waschkolonne eine Waschkolonne mit erzwungenem Transport der Acrylsäurekristalle ist und
c) als Waschflüssigkeit die Schmelze von in der Waschkolonne gereinigten Acrylsäurekristallen verwendet wird.

Grundsätzlich ist das erfindungsgemäße Verfahren für alle in dieser Schrift genannten Rohacrylsäuren geeignet.

Dabei kann in allen Fällen die Erzeugung der Acrylsäurekristalle der Rohacrylsäuresuspension im Beisein von 0,20 bis 10 Gew.-%, oder von 0,40 bis 8 Gew.-%, oder von 0,60 bis 5 Gew.-%, oder von 0,60 bis 3 Gew.-%, oder von 0,60 bis 2 Gew.-%, oder von 0,75 bis 1,5 Gew.-%, bezogen auf das Gewicht der in der Rohacrylsäure enthaltenen Menge an Acrylsäure, an Wasser erfolgen.

Dabei kann die Rohacrylsäure bewußt so hergestellt worden sein , daß sie diese Wassermenge bereits herstellungsbedingt enthält. Üblicherweise ist Rohacrylsäure herstellungsbedingt jedoch im wesentlichen oder vollständig frei an Wasser. In diesen Fällen, oder in Fällen, in denen die herstellungsbedingt in der Rohacrylsäure enthaltene Wassermenge nicht zu befriedigen vermag, kann erfindungsgemäß vor Herstellung der Rohacrylsäuresuspension durch Zusatz von Wasser selbstredend auf den gewünschten Wassergehalt ergänzt werden.

Bei der Herstellung der erfindungsgemäß erforderlichen Rohacrylsäuresuspension können die Feststoffkristalle unmittelbar in Suspension befindlich erzeugt werden. Selbstverständlich können sie aber auch als Schicht auf einer gekühlten Wand abgeschieden werden, von der sie anschließend abgekratzt und in der Restschmelze resuspendiert werden.

D.h., die Feststoffbildung kann erfindungsgemäß in gekühlten Rührkesseln, in Kratzkühlern oder in Scheibenkristallisatoren durchgeführt werden, wie sie z.B. in Chem.-Ing.-Techn. 57 (1985) Nr. 2, S. 91 - 102, beschrieben sind.

Ganz generell kommen für das erfindungsgemäße Verfahren zur Herstellung der Rohacrylsäuresuspension alle Suspensionskristallisatoren in Betracht, die in den in dieser Schrift als Stand der Technik aufgeführten Schriften genannt sind.

Insbesondere können als Suspensionskristallisatoren die nachfolgenden Suspensionskristallisatoren der nachfolgenden Firmen eingesetzt werden:

| | |
|---|---|
| Suspensionskristallisator | Firma |
| Kühlscheibenkristallisator | Goudsche Maschinefabrik BV, (NL) |
| Kühlschabkristallisator | Richard M. Armstrong (Schottland) |
| Zwangsumlaufkristallisator mit außenliegendem Wärmeüberträger | Swenson, Messo Chemietechnik (DE u. SE) |

Die Suspensionskristallisatoren können sowohl im Gleichstrom als auch im Gegenstrom von Kältemittel und Rohacrylsäure betrieben werden. Das letztere ist der Regelfall.

In der Regel weisen die Acrylsäurekristalle der erfindungsgemäß zu erzeugenden Rohacrylsäuresuspension eine quaderförmige Geometrie auf. Die Verhältnisse der Abmessungen der vorgenannten geometrischen Körper sind dabei häufig wie folgt: L(Länge) : B (Breite) : H (Höhe) = 1 bis 5 : 1 :1. Die Länge L liegt häufig bei 10 bis 100 µm, vielfach bei 100 bis 800, beziehungsweise bis 400 µm.

Auf dem Weg vom Kristallisator in die Waschkolonne ist es im Normalfall zweckmäßig, die Kristallsuspension zu homogenisieren (z.B. durch Rühren und/oder geeignete Pumpen).

Als erfindungsgemäß geeignete Waschschmelze-Waschkolonnen können sowohl Hydraulische Waschkolonnen (z.B. jene des Instituts TNO in Apeldoorn, Niederlande (vgl. Applied Thermal Engineering Bd. 17, Nr. 8-10, S. 879-888, 1997, oder Chemical Engineering Science Bd. 50, Nr. 17, S. 2717-2729, 1995, Elsevier Science Ltd., oder 4. BIWIC 94/Bremen International Workshop for Industrial Crystallization, Bremen, 8th-9th September 1994 at the University Bremen, Ed.: J. Ulrich, oder Trans. I. Chem. E, Vol. 72, Part A, Sept. 1994, S. 695 bis 702 und Applied Thermal Engineering Vol. 17, Nos. 8-10, S. 879-888, 1997, Elsevier Science Ltd.)) als auch Mechanische Waschkolonnen (z.B. jene der Fa. Niro, Process Technology B.V., Hertogenbosch, Niederlande) verwendet werden.

Ganz generell können erfindungsgemäß alle jene Waschschmelze-Waschkolonnen mit erzwungenem Transport der Acrylsäurekristalle verwendet werden, die in den in dieser Schrift als Stand der Technik zitierten Schriften genannt sind. Beispielhaft genannt seien die Schriften Chem.-Ing.-Techn. 57 (1985) Nr. 2, S. 91-102 und Chem.-Ing.-Techn. 63 (1991), Nr. 9, S. 881-891 sowie die WO 99/6348.

Erfindungsgemäß günstig sind vor allem auch jene Waschschmelze-Waschkolonnen mit erzwungenem Transport, die in Patenten von TNO bzw. Niro oder anderen Firmen beschrieben sind (vgl. z.B. EP-A 97405, US-A 4735781, WO 00/24491, EP-A 920894, EP-A 398437, EP-A 373720, EP-A 193226, EP-A 191194, WO 98/27240, EP-A 305 316 und US-A 4787985). Dabei ist es erfindungsgemäß unbeachtlich, daß die TNO- und Niro-Systeme häufig primär auf den Entzug von Wasser aus Lebensmittel-Flüssigkeiten oder auf Extraktionen ausgerichtet sind.

Die erfindungsgemäß zu erzeugende Rohacrylsäuresuspension kann mit einem Acrylsäurekristallgehalt, bezogen auf das Gesamtgewicht der Rohacrylsäuresuspension, von 10 bis 80 Gew.-%, häufig 20 bis 60 Gew.-% und vielfach 30 bis 50 Gew.-% erzeugt werden.

Die so erzeugten Rohacrylsäuresuspensionen können entweder als solche oder erst nachdem man einen Teil der in ihnen enthaltenen Restschmelze mechanisch entfernt hat, dem erfindungsgemäßen Waschkolonnenverfahren unterworfen werden. Geeignete Mittel zur mechanischen Separation der Kristallphase sind Pressen, Siebe, Zentrifugen und Filter. Beispielsweise können Bandfilter, Trommelfilter, Seiherschnecken und Bogensiebe angewendet werden. Selbstverständlich eignen sich auch Dekantier- und Sedimentationstechniken. Häufig erfolgt die mechanische Separation der Kristallphase aus der Rohacrylsäuresuspension erfindungsgemäß derart, daß die Kristallphase noch tropfnaß mit Restschmelze behaftet ist. Dabei kann die von der Rohacrylsäuresuspension separierte Acrylsäurekristallphase noch 5 bis 30, beziehungsweise bis 10 Gew.-%, bezogen auf das Gesamtgewicht aus Acrylsäurekristallphase und Restschmelze, an Restschmelze enthalten. Zur erfindungsgemäßen Weiterreinigung solcher tropfnassen Acrylsäurekristallphasen eignen sich insbesondere mechanische Waschschmelze-Waschkolonnen.

Erfindungsgemäß wesentlich ist, daß es zur Erzielung einer befriedigenden Reinigungswirkung nicht erforderlich ist, eine wie vorstehend mechanisch abgetrennte Kristallphase vor ihrer Weiterreinigung in einer Waschschmelze-Waschkolonne mit erzwungenem Transport einer Resuspendierung zu unterwerfen, wie es beispielsweise die WO 98/25889 empfiehlt. Selbstverständlich könnte eine solche Resuspendierung aber vor der Anwendung des erfindungsgemäßen Waschkolonnenreinigungsschrittes durchgeführt werden.

Häufig wird der erfindungsgemäße Waschschmelze-Waschkolonnen-Reinigungsschritt so durchgeführt, daß die Differenz zwischen der Temperatur der der Waschkolonne zugeführten Rohacrylsäuresuspension und der in die Waschkolonne rückgeführten Waschschmelze (die sogenannte Differenztemperatur) 2 bis 15°C, häufig 2 bis 10°C oder 2 bis 4°C beträgt.

Erfindungsgemäß überrascht, daß trotz der sehr viel geringeren Verweilzeit der Acrylsäurekristalle von häufig 5 bis 25, oft 10 bis 15 und vielfach 12 bis 14, beziehgungsweise 2 bis 8 Minuten in den erfindungsgemäß anzuwendenden Waschschmelze-Waschkolonnen mit Zwangstransport eine Reinigungswirkung erzielt wird, die jener in einer Schwerkraft-Waschschmelze-Waschkolonne gleichkommt. Dies wird auf die besondere Kristallbeschaffenheit der Acrylsäurekristalle zurückgeführt, die diese aufgrund ihrer Erzeugung im Beisein von Wasser aufweisen. Darüber hinaus scheint gereinigte Acrylsäure aus solchen Kristallen insbesondere die darin eingeschlossene Essig- und/oder Propionsäure auf besondere und besonders wirksame Weise geradezu herauszuextrahieren, was für den ausgezeichneten Erfolg der erfindungsgemäßen Verfahrensweise gleichfalls ursächlich sein dürfte. Das erfindungsgemäße Verfahren besticht auch dadurch, daß es in hervorragender Weise die niedermolekularen Aldehyde wie Furfurale aus der Rohacrylsäure abzutrennen vermag.

Erfindungsgemäß von Bedeutung ist, daß das erfindungsgemäße Verfahren vorab der Herstellung der Rohacrylsäure keines Zusatzes einer polaren organischen Substanz zur Rohacrylsäure bedarf, wie es die WO 99/06348 als zwingend erforderlich erachtet.

Selbstverständlich kann die erfindungsgemäße Verfahrensweise aber auch mit der Verfahrensweise der WO 99/6348 kombiniert angewendet werden. In diesem Fall erfolgt die Herstellung der Rohacrylsäuresuspension sowohl im Beisein von Wasser als auch nach Zusatz einer polaren organischen Flüssigkeit zur zu reinigenden Rohacrylsäure.

Natürlich kann das erfindungsgemäße Verfahren auch so durchgeführt werden, daß die erfindungsgemäß zu waschende Rohacrylsäuresuspension das Ergebnis einer fraktionierten Kristallisation, z.B. einer fraktionierten Suspensionskristallisation, ist. Erfindungsgemäß wesentlich ist aber, daß eine solche Fraktionierung für einen guten Reinigungserfolg nicht unabdingbar ist.

Selbstverständlich kann die erfindungsgemäß erforderliche Kristallisation durch indirekte Kühlung, z.B. Mantelkühlung und/oder durch direkte Kühlung (z.B. Verwendung eines Kühlmittels wie CO₂ und/oder Propan und/oder Verdampfen von Wasser) bewirkt werden.

Besonders vorteilhaft ist es, das erfindungsgemäße Verfahren auf eine Rohacrylsäure anzuwenden, die nach der in der DE-A 19909923 offenbarten Verfahrensweise erzeugt wird.

Natürlich kann die erfindungsgemäße Verfahrensweise auch mehrfach nacheinander angewendet werden. Auch ist es möglich, die beim erfindungsgemäßen Verfahren der Waschkolonne entnommene Restschmelze (Mutterlauge) in den Prozeß der Rohacrylsäureherstellung, z.B. in die Kolonne der fraktionierten Kondensation des Reaktionsgasgemisches der Gasphasenoxidation, rückzuführen, wie es in der Literatur verschiedentlich empfohlen wird.

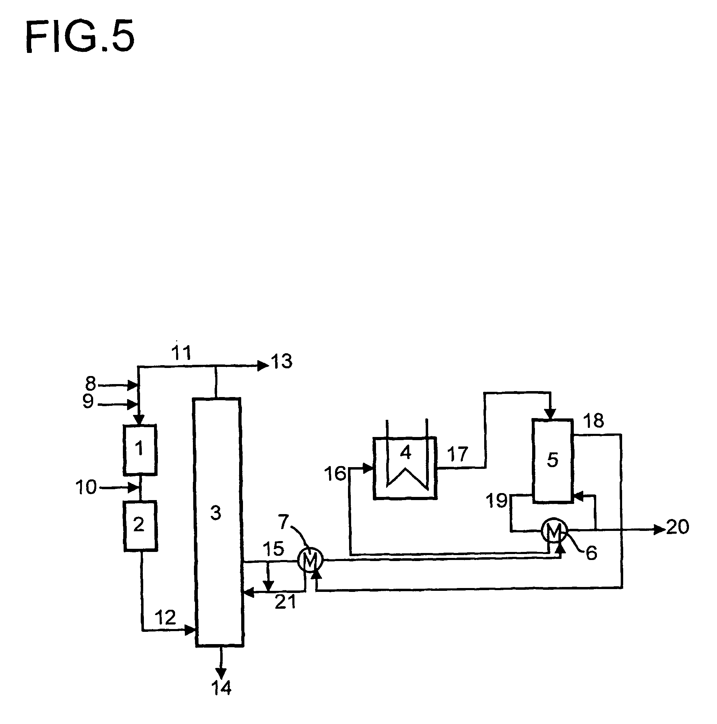

Beispielsweise kann die erfindungsgemäße Verfahrensweise wie folgt in die in den Schriften DE-A 19909923, DE-A 19924533, DE-A 19924532, DE-A 19833049, DE-A 19740253, DE-A 19740252 sowie insbesonder DE-A 19627847 veröffentlichtem Verfahren zur Herstellung von Acrylsäure integriert werden (die schematische Integration zeigt die dieser Schrift beiliegender Figur 5, auf die sichdie nachfolgenden Adressen beziehen).

Danach wird in einer Oxidationszone Propen oder Propan einer heterogen katalytischen Gasphasenoxidation mit molekularem Sauerstoff bei erhöhter Temperatur zu Acrylsäure unterworfen (z.B. werden Propen (8) und Luftsauerstoff (9, 10) in acrylsäurearmes Reaktionskreisgas (11) eingespeist und das Propen in zwei aufeinanderfolgenden Oxidationsstufen (1, 2) an Mo, Bi und Fe (erste Stufe) und an Mo sowie V (zweite Stufe) enthaltenden Multimetalloxidmassen in der Gasphase bei erhöhter Temperatur heterogen katalytisch zu Acrylsäure oxidiert).

Das aus der Oxidationszone kommende, an Acrylsäure angereicherte Reaktionsgas (12) wird (z.B. in einer mehrstufigen Kondensationskolonne (3)) einer fraktionierten Kondensation unterzogen (die Kolonne wird in der Regel adiabat betrieben), bei der eine an Acrylsäure reiche (in typischer Weise 0,2 bis 10 Gew.-% Wasser (bezogen auf enthaltene Acrylsäure) enthaltende) Flüssigfraktion entsteht. Die Einstellung des Wassergehaltes der Rohacrylsäure kann z.B. dadurch erfolgen, daß man vom am Kopf der Kondensationskolonne gewonnenen Sauerwasser innerhalb und/oder außerhalb der Kondensationskolonne zum Entnahmeboden der Rohacrylsäure rückführt.

Diese Rohacrylsäure (15) wird (z.B. über einen Seitenabzug der Kondensationskolonne) entnommen und zur weiteren Reinigung (vorzugsweise ohne vorherigen Zusatz von Fremdsubstanzen) einer Suspensionskristallisationsreinigungsstufe zur weiteren Reinigung zugeführt. Von dem am Kopf der Kondensationskolonne austretenden acrylsäurearmen Gasstrom wird ein Abgasstrom (13) abgetrennt, der neben Luftbestandteilen im wesentlichen Wasser und andere Leichtsieder enthält. Aus dem Sumpf der Kondensationskolonne wird eine schwersiedereiche Flüssigfraktion (14) abgeführt.

Abgasstrom und Schwersiederfraktion können wie in den vorstehend angeführten Schriften beschrieben weiterbehandelt werden. Im Kristallisator (4) werden aus der vorzugsweise vorgekühlten Rohacrylsäure (16) durch Wärmeentzug Acrylsäurekristalle abgeschieden und so eine Suspension erzeugt, die z.B. 20 bis 40 % ihres Gewichtes an Acrylsäurekristallen in verbliebener Mutterlauge enthält.

Diese Suspension (17) wird in unveränderter Form vorzugsweise als solche der relevanten Waschschmelz - Waschkolonne zugeführt, in der durch Filtration und Gegenstromwäsche eine im wesentlichen vollständige Trennung der Acrylsäurekristalle von ihrer Mutterlauge durchgeführt wird.

Die gewaschenen Acrylsäurekristalle werden in einem Schmelzkreislauf (19) aufgeschmolzen. Ein Teil (im Fall einer hydraulischen Waschkolonne in typischer Weise 20 bis 30 Gew.-%) dieser Schmelze wird in der Waschkolonne als Waschmittel für die Gegenstromwäsche verwendet und verlässt in ungünstigen Fällen die Waschkolonne gemeinsam mit der Mutterlauge (18). Der andere Teil der Schmelze wird als vergleichsweise reine Acrylsäure entnommen.

Die Vorkühlung der Rohacrylsäure (15) erfolt zweckmäßig auf indirektem Weg in den Wärmeübertragern (6) und (7). Die dabei abgeführte Wärme wird in vorteilhafter Weise genutzt, um die Acrylsäurekristalle im Schmelzkreis (19, 6) aufzuschmelzen und zweckmäßigerweise in die Kondensationskolonne (3) rückgeführte Mutterlauge (18) vorzuwärmen.

Für die wie beschrieben ein- und austretenden Ströme sind die nachfolgenden Zusammensetzungen typisch:

| | |
|---|---|
| Rohacrylsäure (15/16) | 97,2 Gew.-% Acrylsäure |
| | 4000 Gew.ppm Essigsäure |
| | 619 Gew.ppm Propionsäure |
| | 5000 Gew.ppm Furfural |
| | 703 Gew.ppm Benzaldehyd |
| | 1500 Gew.ppm Maleinsäureanhdydrid |
| | 200 Gew.ppm Phenothiazin |
| | 1,5 Gew.-% Wasser |

| | |
|---|---|
| Mutterlauge (18) | 96,4 Gew.-% Acrylsäure |
| | 6000 Gew.ppm Essigsäure |
| | 744 Gew.ppm Propionsäure |
| | 7000 Gew.ppm Furfural |
| | 925 Gew.ppm Benzaldehyd |
| | 2000 Gew.ppm Maleinsäureanhdydrid |
| | 263 Gew.ppm Phenothiazin |
| | 1,9 Gew.-% Wasser |

| | |
|---|---|
| Gereinigte Acrylsäure | 99,7 Gew.-% Acrylsäure |
| | 1030 Gew.ppm Essigsäure |
| | 225 Gew.ppm Propionsäure |
| | 7 Gew.ppm Furfural |
| | 1 Gew.ppm Benzaldehyd |
| | 2 Gew.ppm Maleinsäureanhdydrid |
| | < 1 Gew.ppm Phenothiazin |
| | 0,1 Gew.-% Wasser. |

D.h., der Verfahrensweg heterogen katalysierte Gasphasenoxidation von Propen oder Propan zu einem Acrylsäure enthaltenden Produktgasgemisch, fraktionierte Kondensation dieses Produktgasgemisches unter Entnahme einer 0,2 bis 10 Gew.-% an Wasser (bezogen auf enthaltene Acrylsäure) enthaltenden Rohacrylsäure, einstufige Suspensionskristallisation der Rohacrylsäure zu einer 20 bis 40 % ihres Gewichtes an Acrylsäurekristallen enthaltenden Kristallsuspension und anschließende erfindungsgemäße Waschung der Kristallsuspension in einer Waschkolonne mit erzwungenem Kristallbetttransport ermöglicht bei minimalem apprativem Aufwand regelmäßig die Herstellung einer Acrylsäurequalität, die ≥ 99,5 % ihres Gewichtes an Acrylsäure enthält.

Als Kälteträger für alle in dieser Schrift angesprochenen indirekten Kühlungen eignen sich Gemische aus Ethylenglykol und Wasser bzw. aus Methanol und Wasser.

Als erfindungsgemäß geeignete Waschkolonnen kommen für die beschriebene Integration, wie bereits gesagt, insbesondere zwei Typen in Betracht:
A) Waschkolonnen mit hydraulischem Transport des Kristallbettes.
B) Waschkolonnen mit mechanischem Transport des Kristallbettes.

Der Kristallvolumenanteil im Kristallbett erreicht in beiden Waschkolonnentypen meist Werte > 0,6. Im Regelfall werden Werte von 0,7 bis 0,75 erreicht. Im folgenden sollen einige dieser Waschkolonnentypen vorgestellt werden.

### A) Geeignete Waschkolonnen mit hydraulischem Transport des Kristallbettes

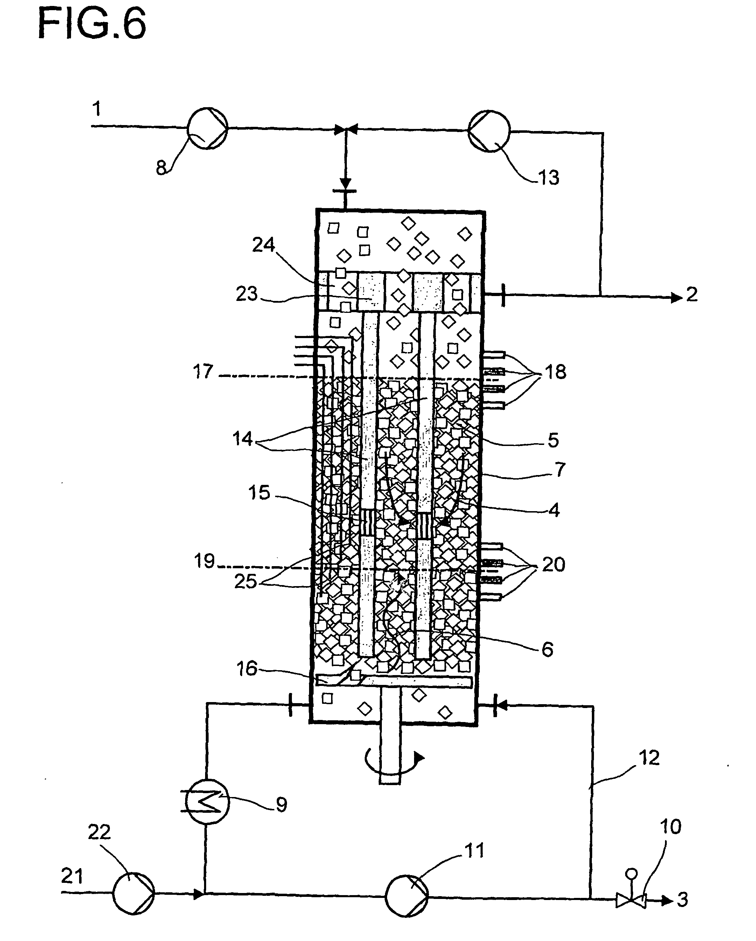

Die dieser Schrift beiliegende Figur 6 zeigt schematisch den Aufbau einer für die beschriebene Integration geeigneten hydraulischen Waschkolonne. Die aus dem Suspensionskristallisator abgezogene Suspension (1) von Acrylsäurekristallen in Mutterlauge wird mittels einer Pumpe (8) und/oder über hydrostatische Höhe unter überatmosphärischem Druck in die Waschkolonne (7) eingespeist. Im oberen Teil der Waschkolonne ist ein Fluidregister angeordnet, das zwei Funktionen erfüllt. Über Durchgangsöffnungen (24) vom oberen zum unteren Kolonnenteil wird die Suspension über den Querschnitt der Waschkolonne verteilt. Der zusammenhängende Innenraum des Fluidregisters (23) dient als Sammler für die abgeführten Flüssigkeiten (Mutterlauge und Waschflüssigkeit (2)). Unten sind am Fluidregister Drainagerohre (14) angebracht (sie weisen innerhalb der Konzentrierungszone einen konstanten Querschnitt auf; das ist aus Sicht der Suspensionszufuhr die Zone bis zum ersten Filter), die mit dem Innenraum (23) verbunden sind. Die Drainagerohre sind in einer definierten Höhe mit wenigstens je einem herkömmlichen Filter (15) versehen, durch das die Mutterlauge (4) aus der Waschkolonne abgeführt wird (dabei kann die Mutterlauge unter Normaldruck, Überdruck oder unter reduziertem Druck stehen). Es bildet sich ein kompaktes Kristallbett (5) aus. Das Kristallbett wird durch die aus dem hydraulischen Strömungsdruckverlust der Mutterlauge resultierende Kraft an den Filtern vorbei in die Waschzone unterhalb der Filter transportiert. Die Rückführung eines Teils der Mutterlauge in die Kolonne mittels der Steuerstrompumpe (13) ermöglicht die Regelung dieser Transportkraft. Schwankungen des Kristallgehalts in der zugeführten Suspension oder Änderungen der Kristallgrößenverteilung, die wesentlich den Strömungsdruckverlust beeinflusst, können dadurch kompensiert werden. Erkennbar sind solche Schwankungen durch die Lageänderung der Filtrationsfront (17), die mit optischen Positionsdetektoren (18) bestimmt werden kann.

Am unteren Ende der Waschkolonne werden die Kristalle mittels eines Rotormessers (16) vom Kristallbett abgetragen und in Reinproduktschmelze, die mit p-Methoxyphenol (MEHQ) überinhibiert sein kann, resuspendiert. Diese Suspension wird in einem Schmelzkreislauf (12) über einen Wärmeträger (9) geführt, über den auf indirektem Weg die zum Schmelzen der Kristalle erforderliche Wärme eingetragen wird. Etwa 70 bis 80 Gew.-%, in günstigen Fällen (z.B. bei ausgeprägter Rekristallisation) sogar > 80 bis 100 Gew.-% der geschmolzenen Kristalle werden als Reinprodukt (3) aus dem Schmelzkreislauf abgeführt. Die Einstellung der entnommenen Menge an Reinprodukt erfolgt über das Produktregelventil (10).

Der verbleibende Teil der Produktschmelze strömt als Waschmittel (6) entgegen der Transportrichtung des Kristallbettes zu den Filtern (15), wodurch in der Waschzone eine Gegenstromwäsche der Kristalle erfolgt. Die Reinigung der Kristalle beruht im wesentlichen auf der Verdrängung und Verdünnung der Mutterlauge in den Zwickeln des Kristallbettes durch Waschflüssigkeit. Der Verdünnungseffekt beruht hierbei auf Vermischung in den durchströmten Zwickeln zwischen den Kristallen und Diffusion in den nicht durchströmten Kontaktstellen, bzw. der oberflächennahen Strömungsgrenzschicht der Kristalle.

Bei stationärem Betrieb stellt sich in einer definierten Höhe in der Waschzone die sogenannte Waschfront (19) ein. Auf der Höhe der Waschfront findet der Konzentrationsübergang von Mutterlaugenkonzentration (oberhalb der Waschfront) und Reinschmelzekonzentration (unterhalb der Waschfront) statt. Die Waschfront (19) muß zur Erzielung einer adäquaten Reinigungswirkung in einer Mindesthöhe oberhalb des Rotormessers (16) positioniert sein. Die Position (19) stellt sich als dynamisches Gleichgewicht aus transportiertem Kristallmassenstrom (5) und entgegengeführtem Waschmittelstrom (6) ein. Die Waschmittelmenge resultiert aus der abgeführten Menge an Reinprodukt.

Bei bereits vergleichsweise guter Reinheit der Rohacrylsäure liegt die Kristallisationstemperatur im Suspensionskristallisator lediglich 3 bis 4°K unterhalb des Reinprodukt-Schmelzpunktes. Im Bereich der Waschfront kommt es daher beim Temperaturausgleich der kalten Kristalle mit der Waschflüssigkeit nur in geringem Umfang zur Rekristallisation der Waschflüssigkeit. Dies begrenzt die Wiedergewinnung an Waschschmelze durch Rekristallisation ebenso wie die Minderung der Porosität des Kristallbettes unterhalb der Waschfront durch Rekristallisation. Eine solche geringe Kristallbettporosität würde den Waschmittelaufwand ebenso verringern wie eine Wiedergewinnung durch Rekristallisation.

Bei guter Reinheit der Rohacrylsäure ist es ferner zweckmäßig, bereits in den Schmelzkreis (12) der Waschkolonne den Lagerstabilisator Methoxyphenol (MEHQ) einzuspeisen. Dazu wird das MEHQ in Reinprodukt gelöst mit einer Dosierpumpe (22) in den relativ warmen Schmelzkreis zur Stabilisierung desselben gegeben. Mit der abgeführten Mischung aus Mutterlauge und Waschschmelze (2) gelangt MEHQ in die zur fraktionierten Kondensation verwendete Kolonne (Fig. 5, Ziffer 3) und stabilisiert diese.

Zur Gewährleistung eines stabilen Betriebs der hydraulischen Waschkolonne im Sinne einer definierten Raum-Zeit-Ausbeute und einer konstant guten Reinigungswirkung ist die Kompensation äußerer Störgrößen wie
- Schwankungen der Suspensionsmenge,
- Änderung des Kristallgehalts in der Suspension,
- Variation der Kristallgrössenverteilung und
- Konzentrationsschwankungen im Feed und/oder der Mutterlauge
durch die Regelung
a) der Filtrationsfront (Fig. 6, Ziffer 17),
b) der spezifischen Waschmittelmenge (Fig. 6, Ziffer 6) und
c) der Schmelzwärme (Fig. 6, Ziffer 12)
zweckmäßig.

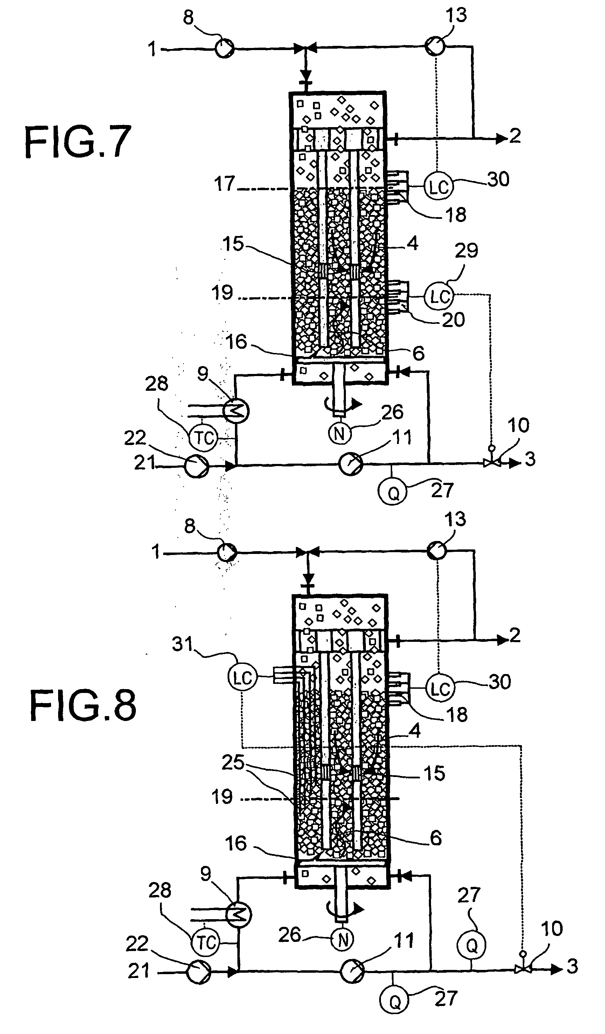

### a) Regelung der Filtrationsfront (die verwendeten Adressen beziehen sich auf die dieser Schrift beiliegenden Figur 7)

Eine konstante Position der Filtrationsfront gewährleistet zu jedem Zeitpunkt die Einhaltung der äußeren Massenbilanz der Waschkolonne. Ihre Position wird bevorzugt mit vier optischen Remissionssensoren (18), die in definierten Höhen in der Kolonnenwand angebracht sind, ermittelt. Als weitere denkbare Detektionsmethoden kommen die Lageerkennnung mittels einer Zeilenkamera durch ein entsprechendes Fenster in der Kolonnenwand oder radiometrische Remissionsverfahren in Betracht. Die Remissionsverfahren beruhen darauf, daß die Intensität der reflektierten Strahlung von der Lage der Bettkante abhängt. Die Zeilenkamera zeigt in einer vertikalen Zeile die komplette Konzentrations- und Waschzone. Die Filtrationsfront zeigt sich durch eine Intensitätsänderung im Zeilensignal. Geregelt (30) wird die rückgeführte Mutterlaugenmenge, die mit der Steuerstrompumpe (13) z.B. durch Drehzahländerung variiert werden kann. Steigt das Kristallbett an, wird die Steuerstrommenge erhöht (dadurch erhöht sich der Druckverlust), bei absinkendem Bett wird sie reduziert. Die Änderung der Steuerstrommenge wird dabei bevorzugt nicht abrupt sonder stetig, z.B. linear über die Zeit, durchgeführt.

### b) Regelung der spezifischen Waschmittelmenge (der Waschfront)

Die spezifische Waschmittelmenge ist die auf den die Waschkolonne effektiv verlassenden Reinproduktstrom bezogene, zur Erzielung einer definierten Trennwirkung aufzuwendende, Waschmittelmenge. Die nachfolgenden Adressen beziehen sich auf die dieser Schrift beiliegenden Figuren 7 bis 9.
Regelkonzept 1:
Einstellung der Waschfront (19) unterhalb des Filters (15)

Die Waschfront (19) wird durch Regelung (Ziffer 29 in Fig. 7 bzw. Ziffer 31 in Fig. 8) der Waschmittelmenge über das Produktventil (10) auf eine definierte Position zwischen Filter (15) und Rotormesser eingestellt. Bei dieser Vorgehensweise wird die Trennaufgabe mit minimalem Waschmittelaufwand erfüllt.

Die Detektion der Waschfront kann z.B. mit vier oder mehr optischen Remissionssensoren (Fig. 7, Ziffer 20) oder bevorzugt mit vier oder mehr im Kristallbett angeordneten Temperaturfühlern (Fig. 8, Ziffer 25) erfolgen.
Regelkonzept 2:
Aussenbilanzierung der spezifischen Waschmittelmenge

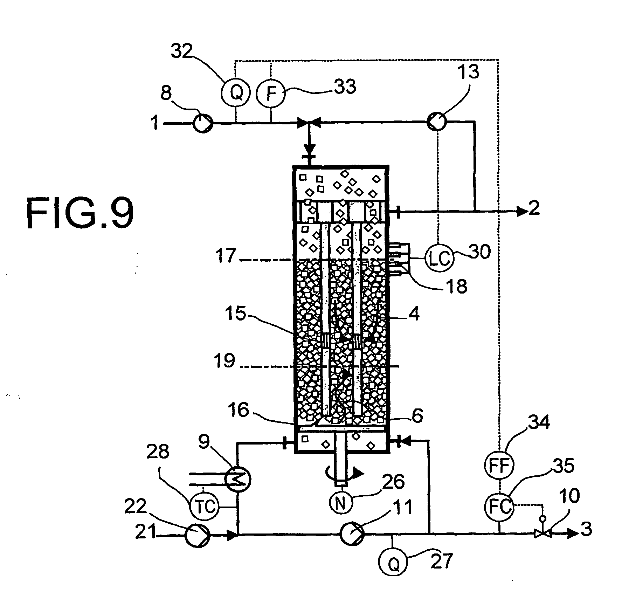

Die spezifische Waschmittelmenge wird in einem definierten, empirisch zu ermittelnden, Verhältnis zur zugeführten Kristallisatmenge eingestellt. Das Verhältnis muß dabei so groß gewählt sein, daß garantiert eine Waschfront aufgebaut wird. Dies gelingt durch Einstellung eines Waschmittelüberschusses (bezogen auf Regelkonzept 1). Die Waschfront (19) stellt sich dabei am Filter (im Bereich von Filterunterkante bis Filtermitte) ein (siehe Fig. 9, auf die sich auch die nachfolgenden Adressen beziehen). Die Regelung der Mengenverhältnisse (34) basiert auf der Messung des Suspensionsmassenstroms (33), des Kristallgehalts in der Suspension (32) und der abgeführten Menge an Roh-Acrylsäure. Eingestellt wird die Waschmittelmenge indirekt über die abgeführte Menge an Reinproduktacrylsäure (33) = Kristallmenge - Waschmittelmenge. Zur Einhaltung und Kontrolle der Reinigungswirkung kann z.B. eine Qualität (27) der Reinproduktacrylsäure verfolgt werden. Die Messung kann dazu mittels eines optischen Extinktionssensors im Spektralbereich 450 nm unmittelbar in der Produktleitung oder im Bypass erfolgen (sie detektiert eine vermutlich auf noch enthaltenes Phenothiazin zurückgehende Färbung). Die Qualitätsmessung erfolgt in der Schmelzkreisleitung und kann dadurch auch für das Anfahren der Waschkolonne genutzt werden. Das Regelkonzept 2 ist einfacher zu praktizieren, weist aber im Vergleich zu Regelkonzept 1 einen erhöhten Waschmittelbedarf auf.

### c) Regelung der Schmelzwärme (die Adressen beziehen sich auf die Figuren 7 bis 9)

Die Eintragung der richtigen Wärmemenge in den Schmelzkreislauf zum Schmelzen der Kristalle wird durch Regelung der Temperatur der Reinproduktacrylsäure (28) nach dem Wärmeübertrager (9) sichergestellt. Die Temperatur im Schmelzkreis kann dabei ca. 1 bis 5°K über dem Schmelzpunkt der Reinproduktacrylsäure liegen.

Das Rotormesser (26) wird zweckmäßig mit einer festen Drehzahl (20 bis 60 Umdrehungen pro Minute) betrieben.

Nähere Ausführungen zur Regelung der Filtrationsfront mit einer Zeilenkamera sowie zur Außenbilanzregelung der spezifischen Waschmittelmenge in einer hydraulischen Waschkolonne finden sich in DE-A 10 036 880 sowie in DE-A 10 036 881.

Die sonstigen beim Betrieb einer defintionsgemäßen hydraulischen Waschkolonne in Betracht zu ziehenden Front- und Schmelzwärmeregelungen sind z.B. in Trans I Chem E, Vol. 72, Part A, September 1994 sowie in den Dissertationen "Hydraulic Wash Columns, Solid-Liquid Separation in Melt Crystallization", Proefschrift von Lianne van Oord-Knol, Technische Universiteit Delft, 13.06.2000 (ISBN 90-805709-1-5) bzw. "Fractional Suspension Crystallization of Organic Compounds", Proefschrift von Pieter Johannes Jansens, Technische Universiteit Delft, 05.04.1994 (ISBN 90-370-0097-5) ausführlich beschrieben.

Anfahrprozedur der hydraulischen Waschkolonne (die Adressen beziehen sich auf Figur 6)

Die aus dem Kristallisator kommende Suspension (1) wird mittels einer Pumpe (8) oder durch hydrostatischen Druck unter atmosphärischem Überdruck in die Waschkolonne (7) eingespeist. In der Waschkolonne sind ein oder mehrere Drainagerohre (14) angeordnet, die in einer definierten Höhe mit wenigstens je einem Filter (15) versehen sind, durch das die Mutterlauge (4) aus der Waschkolonne abgeführt wird. Beim Anfahren der Waschkolonne wird zunächst nur Mutterlauge abgeführt und die in der Waschkolonne verbleibenden Kristalle bilden ein Festbett aus, dessen Porosität ca. 30 bis 40 Vol.-% beträgt. Die Poren zwischen den Kristallen sind vollständig mit Mutterlauge ausgefüllt. Ist eine definierte Betthöhe (17) erreicht, wird das am unteren Ende der Waschkolonne angeordnete Rotormesser (16) eingeschaltet, dessen Aufgabe die gleichmäßige Abnahme des Kristallbetts an seinem unter Ende ist. Die abgeschabten Kristalle gelangen unterhalb des Rotormessers in einen Schmelzkreislauf (12), in dem eine Kreislaufpumpe (11) und ein Wärmeübertrager (9) angeordnet sind. Über den Wärmeüberträger (9) wird die zum Aufschmelzen der Kristalle erforderliche Wärme zugeführt. Um das für den Aufschmelzvorgang erforderliche treibende Temperaturgefälle zu gewährleisten, wird die Produkttemperatur am Austritt des Wärmeüberträgers (9) auf ca. 1 bis 5°K über den Schmelzpunkt des Reinproduktes eingeregelt. Die Schmelze stellt zunächst eine Mischung aus geschmolzenen Kristallen und Mutterlauge dar, mit einem Mischungsverhältnis entsprechend der Porosität des Kristallbetts. Das Produktregelventil (10) bleibt zunächst geschlossen und da stetig Suspension zugespeist wird, strömt die spezifisch leichtere Schmelze (6) zwangsweise entgegen dem nach unten bewegten Kristallbett Richtung Filter. Hierdurch findet eine Waschung der Kristalle statt.

Die in den Poren des Kristallbettes transportierte unreine Mutterlauge wird im Gegenstrom von der reineren Schmelze ausgewaschen und die Waschflüssigkeit (6) entweicht durch das Filter (15). Hierdurch steigert sich nun stetig die Reinheit des Kristallbettes in der Waschzone, dem Bereich zwischen dem Rotormesser (16) und dem Filter (15). Ist die Produktreinheit (Messung vgl. Fig. 7, Ziffer 27) auf ihrem höchsten Niveau angelangt, wird das Produktregelventil soweit geöffnet, dass ca. 70 bis 80 Gew.-%, in günstigen Fällen > 80 bis 100 Gew.-%, der Schmelze die Waschkolonne als Reinprodukt verlassen und nur noch der verbleibende Teil (häufig ca. 20 bis 30 Gew.-%) wie beschrieben als Waschmittel (6) eingesetzt wird und zum Filter hin fließt. Die Waschkolonne befindet sich nun in einem stationären Betriebszustand.

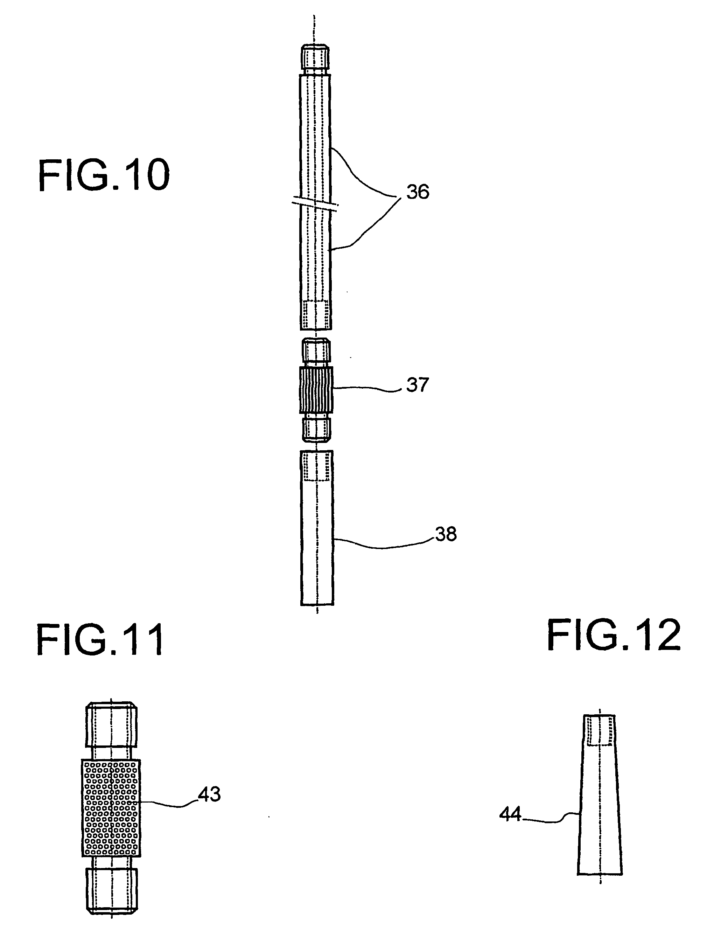

Ausführung der Drainagerohre (siehe beiliegende Figuren 10 und 11)

Die Drainagerohre dienen der Abführung der Mutterlauge und der Waschflüssigkeit aus dem Kristallbett. Alle Drainagerohre in der hydraulischen Waschkolonne sind üblicherweise in gleicher Weise ausgeführt. Sie sind zweckmäßig aus mehreren Bauelementen zusammengesetzt, die folgende Funktionen erfüllen:
- Flüssigkeitstransport
- Filtrieren
- Wärmeisolation
- Wärmeleitung
- Verdrängung

Das Drainagerohr besteht in einer einfachen Version (Figur 10) aus dem Ablaufrohr (36), das die am Filter (37) aus dem Kristallbett aufgenommene Mutterlauge und Waschflüssigkeit zum Fluidregister (siehe Ziffer 23 in Figur 6) abführt und dem Verdränger (38), der dazu dient, die Querschnittsstruktur des Kristallbetts nach dem Filter (37) aufrecht zu erhalten. Die Waschfront befindet sich im stationären Betrieb auf einer Höhe unterhalb des Filters (37) im Bereich des Verdrängers (38). Um eine Abkühlung des Verdrängers (38) unterhalb der Waschfront, durch Wärmeleitung vom warmen Bereich unterhalb der Waschfront in den kalten Bereich oberhalb der Waschfront, zu unterdrücken, ist dieser vorzugsweise aus einem wärmeisolierenden Material wie z.B. Teflon ausgeführt. Dadurch wird verhindert, daß der Verdränger (38) auf eine Temperatur unterhalb der Schmelztemperatur des Waschmittels (= Reinprodukt) abkühlen und das Waschmittel unterhalb der Waschfront am Verdränger (38) kristallisieren kann.

Grundsätzlich können die Filterelemente der Drainagerohre wie in Fig. 10 und 11 dargestellt (37, 39, 41) als Spaltsiebfilter oder als Lochsiebfilter (siehe Figur 11, Ziffer 43) ausgeführt sein.

Bilden die Kristalle im Kristallbett eine für die Waschflüssigkeit schwer zugängliche Zwickelstruktur, kann es vorteilhaft sein, den Verdränger wie in Fig. 12, Ziffer 44 dargestellt, nach unten konisch erweitert zu gestalten. Durch diese Querschnittsänderung entlang des Weges des Kristallbetts, erfährt dieses eine zunehmende Scherspannung, durch die es zu einer Relativbewegung der Kristalle und damit einer Freilegung der Kristallzwickel für die Waschflüssigkeit kommt. Ansonsten weisen die Drainagerohre in der Regel einen konstanten Querschnitt auf.

Die Filterlänge beträgt im Normalfall das 1- bis 3-fache des Drainagerohrdurchmessers. Die Konzentrierungszone des Drainagerohres hat häufig eine Länge von 20 bis 50 cm. Die Länge der Waschzone liegt in der Regel beim 3- bis 10-fachen des Abstandes des Drainagerohres zur Einhüllenden der Waschkolonne.

Im übrigen besitzen die Adressen in den dieser Schrift beiliegenden Figuren 5 bis 9 die nachfolgende Bedeutung:
Fig. 5:
   - 1 =: Oxidationsstufe 1
   - 2 =: Oxidationsstufe 2
   - 3 =: Kondensationskolonne
   - 4 =: Suspensionskristallisator
   - 5 =: hydraulische Waschkolonne
   - 6 =: Aufschmelzwärmeüberträger
   - 7 =: Vorkühler Rohacrylsäure bzw. Vorwärmer rückgeführte Mutterlauge
   - 8 =: Propenzufuhr
   - 9 =: Luftzufuhr 1
   - 10 =: Luftzufuhr 2
   - 11 =: an Acrylsäure armes Kreisgas
   - 12 =: an Acrylsäure reiches Kreisgas
   - 13 =: Abgas
   - 14 =: Schwersiederauslass
   - 15 =: Seitenabzug Rohacrylsäure
   - 16 =: vorgekühlte Rohacrylsäure zur Suspensionskristallisation
   - 17 =: Kristallsuspension zur hydraulischen Waschkolonne
   - 18 =: Mutterlaugeauslaß
   - 19 =: Schmelzkreislauf Reinproduktacrylsäure der hydraulischen Waschkolonne
   - 20 =: Reinproduktacrylsäure-Entnahme
   - 21 =: vorgewärmte Rückfuhr-Mutterlauge
Fig. 6:
   - 1 =: Zufuhr der Kristallisatsuspension
   - 2 =: Mutterlaugeentnahme
   - 3 =: Reinproduktacrylsäure
   - 4 =: interner Mutterlaugestrom
   - 5 =: bewegtes Kristallbett
   - 6 =: Waschschmelze
   - 7 =: Waschkolonne
   - 8 =: Suspensionspumpe
   - 9 =: Wärmeübertrager zum Schmelzen der Kristalle
   - 10 =: Regelventil zur Einstellung des Mengenverhältnisses Waschschmelze/Reinproduktacrylsäure-Entnahme
   - 11 =: Umlaufpumpe des Schmelzkreislaufs
   - 12 =: Schmelzkreislauf
   - 13 =: Steuerstrompumpe
   - 14 =: Drainagerohr für Mutterlauge und Waschflüssigkeit
   - 15 =: Filter
   - 16 =: Rotormesser zur Resuspendierung der gewaschenen Kristalle
   - 17 =: Filtrationsfront (Obergrenze Kristallbett)
   - 18 =: Detektion der Filtrationsfront (4 optische Remissionssensoren)
   - 19 =: Waschfront (Konzentrationsübergang reine-unreine Flüssigphase)
   - 20 =: Detektion der Waschfront (4 optische Remmissionssensoren)
   - 21 =: Inhibitorlösung (MEHQ in Reinproduktacrylsäure)
   - 22 =: Dosierpumpe für die Inhibitorlösung
   - 23 =: Fluidregister: Sammelboden für Mutterlauge und Waschflüssigkeit
   - 24 =: Fluidregister: Verteilerboden für die Kristallisatsuspension
   - 25 =: Detektion der Waschfront (4 Temperaturfühler)
Fig. 7 und 8:
   Wie bei Fig. 6, sowie zusätzlich:
   - 26 =: Drehzahl Rotormesser (10 bis 60 Upm)
   - 27 =: Qualitätskontrolle der Reinproduktacrylsäure (optische Extinktionsmessung bei 450 nm)
   - 28 =: Temperaturregelung im Schmelzkreislauf (14 bis 25°C)
   - 29 =: 4-Punkt Positionsregelung der Waschfront (optische Positionsdetektion)
   - 30 =: 4-Punkt Positionsregelung der Filtrationsfront (optische Positionsdetektion)
   - 31 =: 4-Punkt Positionsregelung der Waschfront (thermische Positionsdetektion)
Fig. 9:
   Wie bei Fig. 7 und 9, sowie zusätzlich:
   - 32 =: Messung der Kristallisatsuspensionsdichte (Kristallisatanteil der Suspension)
   - 33 =: Messung des Suspensionsmassenstroms
   - 34 =: Verhältnisregelung (Masse Waschmittel = Faktor x Masse Kristallisatentnahme)
   - 35 =: Regelung des Waschmittelmassenstroms; Sollwert von 34
Fig. 10 bis 12:
   - 36 =: Ablaufrohr
   - 37 =: Filter für Mutterlauge und Waschmittel; hier als Spaltfilter ausgeführt
   - 38 =: Verdränger aus wärmeisolierendem Material (z.B. Teflon); hier zylindrisch ausgeführt
   - 43 =: Filter mit Lochgeometrie ausgeführt
   - 44 =: Verdränger in konischer Ausführung

Mit Vorteil wird die beschriebene hydraulische Waschkolonne in Anwendung pulsierender Flüssigkeitsströme betrieben. Das sind Ströme, deren Betrag der Fließgeschwindigkeit, aber nicht die Fließrichtung, periodisch über die Zeit varriert. Die Fließrichtung wird zu keinem Zeitpunkt umgekehrt. Sie sind in einfacher Weise z.B. dadurch zu realisieren, daß man entweder den Betrag der Fließgeschwindigkeit des Stroms der der Waschkolonne zugeführten Waschschmelze oder den Betrag der Fließgeschwindigkeit des Stroms der der Waschkolonne entnommenen Mutterlauge über die Zeit periodisch varriert. Auch kann der Betrag der Fließgeschwindigkeit des Stroms der der Waschkolonne zugeführten Suspension periodisch über die Zeit varriert werden.

### B) Geeignete Waschkolonnen mit mechanischem Transport des Kristallbettes

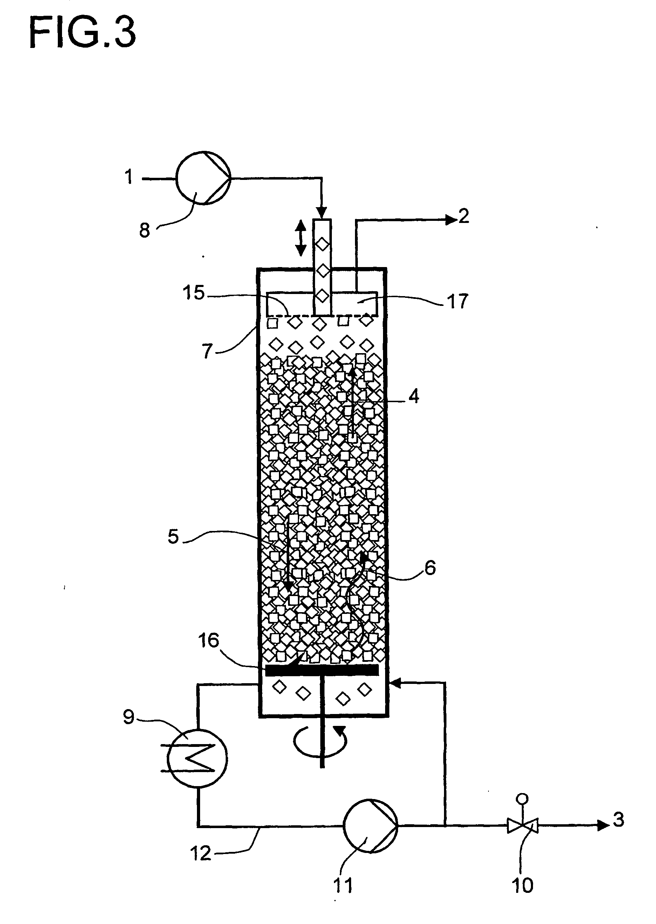

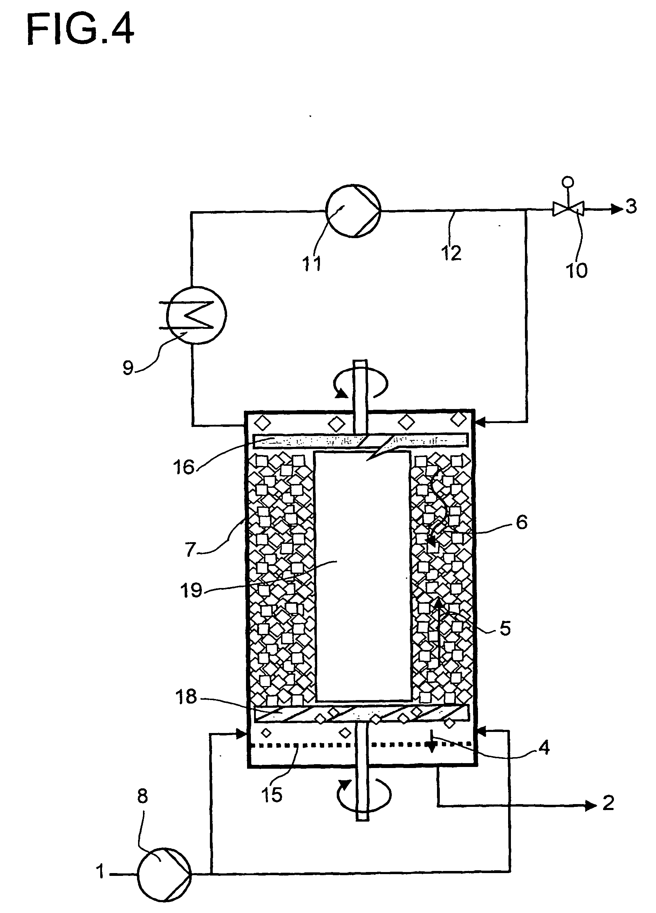

Für die beschriebene Integration geeignete Waschkolonnen mit mechanischem Transport des Kristallbettes unterscheiden sich von hydraulischen Waschkolonnen im wesentlichen dadurch, daß der Transport des Kristallbettes durch eine mechanische Vorrichtung (z.B. ein Schrägblattrotor oder ein oszillierender Kolben) bewirkt wird, weshalb sie keine Drainagerohre enthalten. Das konventionelle Filter zur Abtrennung der Mutterlauge befindet sich normalerweise entweder in der mechanischen Fördereinrichtung (z.B. im Fall des oszillierenden Kolben) oder hinter der mechanischen Fördereinrichtung (z.B. im Fall des Schrägblattrotors). Schematische Darstellungen von für die beschriebene Integration geeigneten mechanischen Waschkolonnen zeigen die dieser Patentlage als Anlage beiliegenden Fig. 3 und 4.

Als Material empfiehlt sich für die erfindungsgemäß einzusetzenden Waschkolonnen Edelstahl, insbesondere Edelstahl der Sorte 1.4571. Dies gilt auch für die Filter.

Die der Waschkolonne entnommene Reinschmelze wird in an sich bekannter Weise durch Zusatz von Polymerisationsinhibitoren polymerisationsinhibiert. Bei besonders hoher Reinheit der in der Rohacrylsäuresuspension befindlichen Acrylsäurekristalle wird bereits zu deren Aufschmelzen in an sich bekannter Weise Polymerisationsinhibitor zugesetzt. Dies kann z.B so erfolgen, daß der Monomethylether des Hydrochinons in Reinproduktschmelze (Acrylsäure) gelöst wird (z.B. in einem Gewichtsanteil von bis zu 1000 Gew.ppm oder mehr, bezogen auf die Lösung) und diese Lösung zum Aufschmelzen zugesetzt wird (vgl. z.B. EP-A 776875).

Der geringste Porositätsgrad innerhalb des Kristallbetts in der findungsgemäß zu verwendenden Waschschmelze-Waschkolonne beträgt im Normalfall ≤ 0,45, häufig 0,15 bis 0,35.

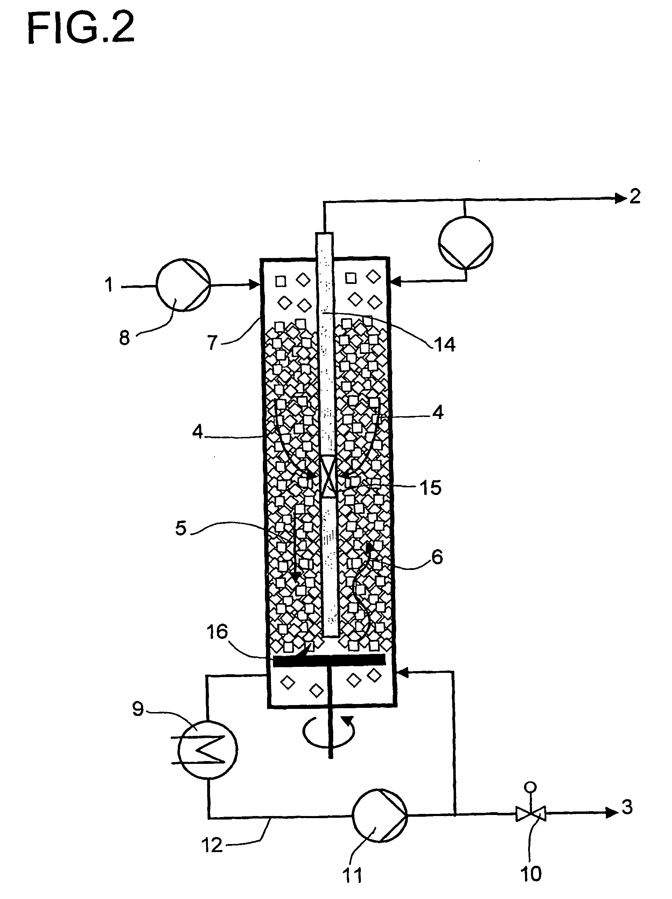

Wird erfindungsgemäß eine hydraulische Waschschmelze-Waschkolonne eingesetzt (geeignet wäre, wie bereits gesagt, z.B. eine solche gemäß EP-A 398 437, EP-A 97405 bzw. US-A 4,735,781), liegt der hydraulische Druck in der Regel bei 0,1 bis 10 bar, häufig bei 1 bis 3 bar. Erfindungsgemäß können auch gepulste Waschkolonnen eingesetzt bzw. die Waschkolonne mit pulsierenden Strömen betrieben werden, wie es z.B. die EP-A 97405 beschreibt. Prinzipienskizzen einiger erfindungsgemäß geeigneter Waschschmelze-Waschkolonnen zeigen, wie bereits gesagt, die Figuren 2 bis 4 (Fig. 2 = hydraulischer Kristallbett-Transport, Fig. 3,4 = mechanischer Bett-Transport und Fig. 1 = gravitativer Bett-Transport).

Dabei haben die Ziffern dieser Figuren die nachfolgende Bedeutung:
- 1:: Suspension
- 2:: Restschmelze (Mutterlauge)
- 3:: Produkt (geschmolzenes Reinkristallisat)
- 4:: Unreine Restschmelze
- 5:: Bewegtes Kristallbett
- 6:: Waschflüssigkeit(schmelze)
- 7:: Waschkolonne
- 8:: Suspensionspumpe
- 9:: Wärmeübertrager zum Schmelzen der Kristalle
- 10:: Regelventil zum Einstellen des Mengenverhältnisses Waschflüssigkeit(schmelze)/Produkt
- 11:: Umlaufpumpe des Schmelzkreislaufs
- 12:: Schmelzkreislauf
- 13:: Rührer
- 14:: Filterrohr
- 15:: Filter
- 16:: Rotierendes Messer zur Resuspendierung der gewaschenen Kristalle
- 17:: Oszillierender Kolben mit filtrierender Stirnfläche und Restschmelzeablauf
- 18:: Schrägblattrotor für den Transport des Kristallbetts
- 19:: Zylindrischer Verdränger

### Beispiele:

Analog zu Beispiel 1 der DE-A 19909923 werden 150 kg/h einer Rohacrylsäure der nachfolgenden Zusammensetzung erzeugt:

| | |
|---|---|
| Acrylsäure | 98,5 Gew.-% |
| Essigsäure | 0,8 Gew.-% |
| Propionsäure | 500 Gew.-ppm |
| Furfural | 4800 Gew.-ppm |
| Maleinsäureanhydrid | 40 Gew.-ppm |
| Benzaldehyd | 680 Gew.-ppm |
| Wasser | 1,5 Gew.-% |
| Phenothiazin | 200 Gew.-ppm |

Die Rohacrylsäure wird einem Suspensionskristallisator zugeführt. Der Suspensionskristallisator ist ein Kühlscheibenkristallisator (100 1 Innenvolumen). Die Kristallisationswärme wird über die Kühlflächen des Behälters abgeführt. Die Gleichgewichtstemperatur der Restschmelze beträgt 9,5°C. Die bei der Kristallisation erzeugte Rohracrylsäuresuspension (Feststoffgehalt ca. 20 Gew.-%) wird in voneinander unabhängigen Versuchen
a) in einer Schwerkraft-Waschschmelze-Waschkolonne gewaschen;
b) in einer hydraulischen Waschschmelze-Waschkolonne gewaschen;
c) auf einer Zentrifuge (800-fache Erdbeschleunigung) bei einer Schleuderzeit von 30 sec diskontinuierlich in Kristalle und Mutterlauge getrennt. Die Kristalle werden anschließend mit aufgeschmolzenem (zuvor gewaschenem) Kristallisat (im Massenverhältnis "1 Teil Waschmittel auf 5 Teile Kristallisat") 30 sec lang bei 800-facher Erdbeschleunigung gewaschen.
Das Rücklaufverhältnis (Verhältnis von je Zeiteinheit entnommener Reinschmelzemenge zu je Zeiteinheit rückgeführter Waschschmelzmenge) und die Differenztemperatur der Waschkolonnen werden ca. identisch gewählt. Die Analyse der gewaschenen Kristalle ergibt für die Fälle a) bis c):

| c) : | | a) und b) : | |
|---|---|---|---|
| Acrylsäure | 99,6 Gew.-% | Acrylsäure | 99,8 |
| Essigsäure | 0,18 Gew.-% | Essigsäure | 1350 Gew.ppm |
| Propionsäure | 203 Gew.-ppm | Propionsäure | 170 Gew.-ppm |
| Furfural | 405 Gew.-ppm | Furfural | 14 Gew.ppm |
| Maleinsäureanhydrid | 1,6 Gew.-ppm | Maleinsäureanhydrid | nicht mehr feststellbar |
| Benzaldehyd | 58 Gew.-ppm | Benzaldehyd | nicht mehr |
| Wasser | 2700 Gew.-ppm | | feststellbar |
| Phenothiazin | 10 Gew.-ppm | Wasser | 200 ppm |
| | | Phenothiazin | nicht mehr detektierbar |
| | | Auf den Wegen a) und b) wird eine bessere Reinheit erhalten als auf dem Weg c); die Ergebnisse längs der Wege a) und b) sind im wesentlichen ununterscheidbar. Insbesondere enthält die längs a) und b) zu gewinnende kristalline Acrylsäure kaum noch Essigsäure. | |

## Patentansprüche

1. Verfahren zur Reinigung einer Rohacrylsäureschmelze, die, bezogen auf ihr Gewicht, in das in der Rohacrylsäureschmelze enthaltenes Wasser nicht mit eingerechnet wird,
≥ 80 Gew.-% Acrylsäure
und als von Acrylsäure verschiedene Verunreinigungen wenigstens
≥ 100 Gew.-ppm Essigsäure und
≥ 10 Gew.-ppm Propionsäure
enthält, bei dem man die Rohacrylsäureschmelze unter Einwirkung von Kälte in eine aus Acrylsäurekristallen und Restschmelze bestehende Rohacrylsäuresuspension überführt, wobei der Gewichtsanteil der Acrylsäurekristalle an von Acrylsäure verschiedenen Verunreinigungen kleiner und der Gewichtsanteil der Restschmelze an von Acrylsäure verschiedenen Verunreinigungen größer ist als der Gewichtsanteil der Rohacrylsäureschmelze an von Acrylsäure verschiedenen Verunreinigungen, von der Rohacrylsäuresuspension gegebenenfalls einen Teil der Restschmelze mechanisch abtrennt und die Acrylsäurekristalle der verbliebenen Rohacrylsäuresuspenion in einer Waschkolonne von verbliebener Restschmelze befreit, mit der Maßgabe daß
a) die Erzeugung der Acrylsäurekristalle der Rohacrylsäuresuspension im Beisein von 0,20 bis 10 Gew.-%, bezogen auf das Gewicht der in der Rohacrylsäureschmelze enthaltenen Menge an Acrylsäure, an Wasser erfolgt,
b) die Waschkolonne eine Waschkolonne mit erzwungenem Transport der Acrylsäurekristalle ist und
c) als Waschflüssigkeit die Schmelze von in der Waschkolonne gereinigten Acrylsäurekristallen verwendet wird.

2. Verfahren nach Anspruch 1, **dadurch gekennzeichnet, daß** die Rohacrylsäureschmelze, bezogen auf ihr Gewicht, in das in der Rohacrylsäureschmelze enthaltenes Wasser nicht mit eingerechnet wird,
≥ 80 Gew.-% Acrylsäure,
≥ 100 Gew.-ppm bis ≤ 15 Gew-% Essigsäure,
≥ 10 Gew.-ppm bis ≤ 5 Gew-% Propionsäure,
bis zu 5 Gew.-% niedermolekularen Aldehyde,
bis zu 3 Gew.-% Polymerisationsinhibitoren und
0 bis 5 Gew.-% Acrylsäure-Oligomere
enthält.

3. Verfahren nach Anspruch 1, **dadurch gekennzeichnet, daß** die Rohacrylsäureschmelze, bezogen auf ihr Gewicht, in das in der Rohacrylsäureschmelze enthaltenes Wasser nicht mit eingerechnet wird,
≥ 90 Gew.-% Acrylsäure,
≥ 100 Gew.-ppm bis ≤ 5 Gew-% Essigsäure,
≥ 10 Gew.-ppm bis ≤ 2 Gew-% Propionsäure,
bis zu 2 Gew.-% niedermolekulare Aldehyde,
bis zu 2 Gew.-% Polymerisationsinhibitoren und
0 bis 3 Gew.-% Acrylsäure-Oligomere (Michael-Addukte)
enthält.

4. Verfahren nach Anspruch 1, **dadurch gekennzeichnet, daß** die Rohacrylsäureschmelze, bezogen auf ihr Gewicht, in das in der Rohacrylsäureschmelze enthaltenes Wasser nicht mit eingerechnet wird,
≥ 95 Gew.-% Acrylsäure,
≥ 100 Gew.-ppm bis ≤ 3 Gew-% Essigsäure,
≥ 10 Gew.-ppm bis ≤ 2 Gew-% Propionsäure
bis zu 2 Gew.-% niedermolekulare Aldehyde,
bis zu 2 Gew.-% Polymerisationsinhibitoren und
0 bis 2 Gew.-% Acrylsäure-Oligomere (Michael-Addukte)
enthält.

5. Verfahren nach einem der Ansprüche 1 bis 4, **dadurch gekennzeichnet, daß** die Erzeugung der Acrylsäurekristalle der Rohacrylsäuresuspension im Beisein von 0,40 bis 8 Gew.-%, bezogen auf das Gewicht der in der Rohacrylsäurechmelze enthaltenen Menge an Acrylsäure, an Wasser erfolgt.

6. Verfahren nach einem der Ansprüche 1 bis 4, **dadurch gekennzeichnet, daß** die Erzeugung der Acrylsäurekristalle der Rohacrylsäuresuspension im Beisein von 0,60 bis 5 Gew.-%, bezogen auf das Gewicht der in der Rohacrylsäureschmelze enthaltenen Menge an Acrylsäure, an Wasser erfolgt.

7. Verfahren nach einem der Ansprüche 1 bis 4, **dadurch gekennzeichnet, daß** die Erzeugung der Acrylsäurekristalle der Rohacrylsäuresuspension im Beisein von 0,60 bis 3 Gew.-%, bezogen auf das Gewicht der in der Rohacrylsäureschmelze enthaltenen Menge an Acrylsäure, an Wasser erfolgt.

8. Verfahren nach einem der Ansprüche 1 bis 7, **dadurch gekennzeichnet, daß** die Differenztemperatur der Waschkolonne 2 bis 10°C beträgt.

9. Verfahren nach einem der Ansprüche 1 bis 8, **dadurch gekennzeichnet, daß** eine mechanische Waschkolonne verwendet wird.

10. Verfahren nach einem der Ansprüche 1 bis 8, **dadurch gekennzeichnet, daß** eine hydraulische Waschkolonne verwendet wird.

## Claims

1. A process for purifying a crude acrylic acid melt which contains, based on its weight, which does not include water present in the crude acrylic acid melt,
≥ 80% by weight of acrylic acid
and, as impurities different from acrylic acid, at least
≥ 100 ppm by weight of acetic acid and
≥ 10 ppm by weight of propionic acid,
in which the crude acrylic acid melt is converted, under the action of low temperatures, into a crude acrylic acid suspension consisting of acrylic acid crystals and residual melt, the amount by weight of impurities different from acrylic acid in the acrylic acid crystals being less than, and the amount by weight of impurities different from acrylic acid in the residual melt being greater than, the amount by weight of impurities different from acrylic acid in the crude acrylic acid melt, if necessary a part of the residual melt is separated off mechanically from the crude acrylic acid suspension and the acrylic acid crystals of the remaining crude acrylic acid suspension are freed from remaining residual melt in a wash column, with the proviso that
a) the production of the acrylic acid crystals of the crude acrylic acid suspension is effected in the presence of from 0.20 to 10% by weight, based on the weight of the acrylic acid contained in the crude acrylic acid melt, of water,
b) the wash column is a wash column with forced transport of the acrylic acid crystals and
c) the wash liquid used is the melt of acrylic acid crystals purified in the wash column.

2. A process as claimed in claim 1, wherein the crude acrylic acid melt contains, based on its weight, which does not include the water present in the crude acrylic acid melt,
≥ 80% by weight of acrylic acid,
from ≥ 100 ppm by weight to ≤ 15% by weight of acetic acid,
from ≥ 10 ppm by weight to ≤ 5% by weight of propionic acid,
up to 5% by weight of low molecular weight aldehydes,
up to 3% by weight of polymerization inhibitors and from 0 to 5% by weight of acrylic acid oligomers

3. A process as claimed in claim 1, wherein the crude acrylic acid melt contains, based on its weight, which does not include the water present in the crude acrylic acid melt,
≥ 90% by weight of acrylic acid,
from ≥ 100 ppm by weight to ≤ 5% by weight of acetic acid,
from ≥ 10 ppm by weight to ≤ 2% by weight of propionic acid,
up to 2% by weight of low molecular weight aldehydes,
up to 2% by weight of polymerization inhibitors and
from 0 to 3% by weight of acrylic acid oligomers (Michael adducts).

4. A process as claimed in claim 1, wherein the crude acrylic acid melt contains, based on its weight, which does not include the water present in the crude acrylic acid melt,
≥ 95% by weight of acrylic acid,
from ≥ 100 ppm by weight to ≤ 3% by weight of acetic acid,
from ≥ 10 ppm by weight to ≤ 2% by weight of propionic acid,
up to 2% by weight of low molecular weight aldehydes,
up to 2% by weight of polymerization inhibitors and
from 0 to 2% by weight of acrylic acid oligomers (Michael adducts).

5. A process as claimed in any of claims 1 to 4, wherein the production of the acrylic acid crystals of the crude acrylic acid suspension is effected in the presence of from 0.40 to 8% by weight, based on the weight of the acrylic acid contained in the crude acrylic acid melt, of water.

6. A process as claimed in any of claims 1 to 4, wherein the production of the acrylic acid crystals of the crude acrylic acid suspension is effected in the presence of from 0.60 to 5% by weight, based on the weight of the acrylic acid contained in the crude acrylic acid melt, of water.

7. A process as claimed in any of claims 1 to 4, wherein the production of the acrylic acid crystals of the crude acrylic acid suspension is effected in the presence of from 0.60 to 3% by weight, based on the weight of the acrylic acid contained in the crude acrylic acid melt, of water.

8. A process as claimed in any of claims 1 to 7, wherein the temperature difference of the wash column is from 2 to 10°C.

9. A process as claimed in any of claims 1 to 8, wherein a mechanical wash column is used.

10. A process as claimed in any of claims 1 to 8, wherein a hydraulic wash column is used.

## Revendications

1. Procédé de purification d'une masse fondue d'acide acrylique brut qui, par rapport à son poids dans lequel l'eau contenue dans la masse fondue d'acide acrylique brut n'est pas comptée, contient
≥ 80 % en poids d'acide acrylique,
et, comme impuretés différentes de l'acide acrylique, au moins
≥ 100 ppm en poids d'acide acétique, et
≥ 10 ppm en poids d'acide propionique,
dans lequel, sous l'action d'un froid, on convertit la masse fondue d'acide acrylique brut en une suspension d'acide acrylique brut constituée de cristaux d'acide acrylique et d'une masse fondue résiduaire, la fraction en poids des cristaux d'acide acrylique en impuretés différentes de l'acide acrylique étant plus petite, et la fraction en poids de la masse fondue résiduaire en impuretés différentes de l'acide acrylique étant plus grande que la fraction en poids de la masse fondue d'acide acrylique brut en impuretés différentes de l'acide acrylique, on isole éventuellement mécaniquement de la suspension d'acide acrylique brut une partie de la masse fondue résiduaire et, dans une colonne de lavage, on libère de la masse fondue résiduaire subsistante les cristaux d'acide acrylique de la suspension d'acide acrylique brut subsistante, avec la condition que
a) la production des cristaux d'acide acrylique de la suspension d'acide acrylique brut ait lieu en présence de 0,20 à 10 % en poids d'eau, par rapport au poids de la quantité d'acide acrylique contenue dans la masse fondue d'acide acrylique brut,
b) la colonne de lavage soit une colonne de lavage à transport forcé des cristaux d'acide acrylique, et que
c) la masse fondue de cristaux d'acide acrylique purifiés dans la colonne de lavage soit utilisée comme liquide de lavage.

2. Procédé suivant la revendication 1, **caractérisé en ce que** la masse fondue d'acide acrylique brut contient, par rapport à son poids dans lequel l'eau contenue dans la masse fondue d'acide acrylique brut n'est pas comptée,
≥ 80 % en poids d'acide acrylique,
≥ 100 ppm en poids jusqu'à ≤ 15 % en poids d'acide acétique,
≥ 10 ppm en poids jusqu'à ≤ 5 % en poids d'acide propionique,
jusqu'à 5 % en poids d'aldéhydes de faible poids moléculaire,
jusqu'à 3 % en poids d'inhibiteurs de polymérisation, et
0 à 5 % en poids d'oligomères d'acide acrylique.

3. Procédé suivant la revendication 1, **caractérisé en ce que** la masse fondue d'acide acrylique brut contient, par rapport à son poids dans lequel l'eau contenue dans la masse fondue d'acide acrylique brut n'est pas comptée,
≥ 90 % en poids d'acide acrylique,
≥ 100 ppm en poids jusqu'à ≤ 5 % en poids d'acide acétique,
≥ 10 ppm en poids jusqu'à ≤ 2 % en poids d'acide propionique,
jusqu'à 2 % en poids d'aldéhydes de faible poids moléculaire,
jusqu'à 2 % en poids d'inhibiteurs de polymérisation, et
0 à 3 % en poids d'oligomères d'acide acrylique (produits d'addition de Michael).

4. Procédé suivant la revendication 1, **caractérisé en ce que** la masse fondue d'acide acrylique brut contient, par rapport à son poids dans lequel l'eau contenue dans la masse fondue d'acide acrylique brut n'est pas comptée,
≥ 95 % en poids d'acide acrylique,
≥ 100 ppm en poids jusqu'à ≤ 3 % en poids d'acide acétique,
≥ 10 ppm en poids jusqu'à ≤ 2 % en poids d'acide propionique,
jusqu'à 2 % en poids d'aldéhydes de faible poids moléculaire,
jusqu'à 2 % en poids d'inhibiteurs de polymérisation, et
0 à 2 % en poids d'oligomères d'acide acrylique (produits d'addition de Michael).

5. Procédé suivant l'une des revendications 1 à 4, **caractérisé en ce que** la production des cristaux d'acide acrylique de la suspension d'acide acrylique brut a lieu en présence de 0,40 à 8 % en poids d'eau, par rapport au poids de la quantité d'acide acrylique contenue dans la masse fondue d'acide acrylique brut.

6. Procédé suivant l'une des revendications 1 à 4, **caractérisé en ce que** la production des cristaux d'acide acrylique de la suspension d'acide acrylique brut a lieu en présence de 0,60 à 5 % en poids d'eau, par rapport au poids de la quantité d'acide acrylique contenue dans la masse fondue d'acide acrylique brut.

7. Procédé suivant l'une des revendications 1 à 4, **caractérisé en ce que** la production des cristaux d'acide acrylique de la suspension d'acide acrylique brut a lieu en présence de 0,60 à 3 % en poids d'eau, par rapport au poids de la quantité d'acide acrylique contenue dans la masse fondue d'acide acrylique brut.

8. Procédé suivant l'une des revendications 1 à 7, **caractérisé en ce que** la température différentielle de la colonne de lavage est de 2 à 10°C.

9. Procédé suivant l'une des revendications 1 à 8, **caractérisé en ce qu'**on utilise une colonne de lavage mécanique.

10. Procédé suivant l'une des revendications 1 à 8, **caractérisé en ce qu'**on utilise une colonne de lavage hydraulique.
